(19) Europäisches Patentamt
European Patent Office
Office européen des brevets

(11) Numéro de publication: 0 366 511 A1

(12) # DEMANDE DE BREVET EUROPEEN

(21) Numéro de dépôt: 89402736.6

(22) Date de dépôt: 04.10.89

(51) Int. Cl.5: C07D 263/58 , A61K 31/42 , C07D 413/12

Revendications pour les Etats contractants suivants: ES + GR

(30) Priorité: 04.10.88 FR 8812945

(43) Date de publication de la demande: 02.05.90 Bulletin 90/18

(84) Etats contractants désignés: AT BE CH DE ES FR GB GR IT LI LU NL SE

(71) Demandeur: ADIR ET COMPAGNIE
22, rue Garnier
F-92201 Neuilly sur Seine(FR)

(72) Inventeur: Lesieur, Daniel
20 rue de Verdun
F-59147 Gondecourt(FR)
Inventeur: Abdellatifi, Nourddine
30 résidence le Geai
F-59139 Wattignies(FR)
Inventeur: Aichaoui, Hocine
30 résidence de la Paix Tour Kennedy
F-59120 Loos(FR)
Inventeur: Bonnet, Jacqueline
19 rue Charcot
F-75013 Paris(FR)

(54) **Nouveaux dérivés benzoxazolinoniques, leurs procédés de préparation et les compositions pharmaceutiques qui les contiennent.**

(57) Composés de formule générale (I) :

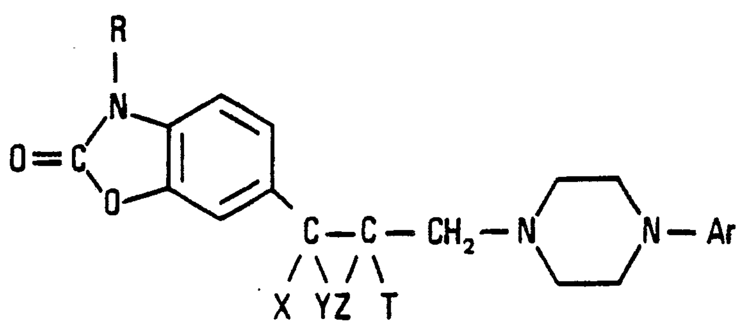

(I)

dans laquelle :
X représente un atome d'hydrogène,
Y représente un atome d'hydrogène ou un groupement hydroxyle ou bien X et Y représentent ensemble un atome d'oxygène,
T représente un atome d'hydrogène ou un groupement alcoyle inférieur,
Z représente un atome d'hydrogène ou bien Z forme avec Y une liaison π , et dans ce cas X représente un atome d'hydrogène,
R représente un atome d'hydrogène ou un groupement alcoyle inférieur,
Ar représente un groupement aryle, hétéroaryle, ou alkyle inférieur aryle tel que pyrimidinyle, éventuellement substitué par un atome d'halogène, un groupement alcoyle ou alcoyloxy inférieur, eux-mêmes éventuellement substitués par un ou plusieurs atomes d'halogène,
leurs énantiomères, épimères, diastéréoisomères,

EP 0 366 511 A1

ainsi que leurs sels d'addition à un acide pharmaceutiquement acceptable.
Médicaments.

# NOUVEAUX DERIVES BENZOXAZOLINONIQUES, LEURS PROCEDES DE PREPARATION ET LES COMPOSITIONS PHARMACEUTIQUES QUI LES CONTIENNENT

La présente invention concerne de nouveaux dérivés de la benzoxazolinone, leurs préparations et les compositions pharmaceutiques qui les contiennent.

De nombreux dérivés de benzoxazolinone ont été décrits en thérapeutique comme possédant des activités pharmacologiques très variées. Le brevet FR 73.23280 décrit des acyl - 6 benzoxazolinones en tant qu'analgésiques. Le brevet FR 80.20861 décrit notamment des (amino - 2 éthyl) - 6 benzoxazolinones et (amino acétyl) - 6 benzoxazolinones utilisables dans le traitement de l'hypertension artérielle ainsi que dans celui des syndrômes douloureux. Le brevet FR 82.19812 décrit des (amino -2 éthyl) - 6 benzoxazolinones utilisables en thérapeutique dans le traitement des troubles du sommeil et des troubles du caractère et du comportement.

La demanderesse a maintenant découvert des dérivés benzoxazolinoniques doués d'une activité analgésique dépourvue d'activité anti-inflammatoire d'un niveau nettement plus intéressant que celui des dérivés décrits dans les brevets FR 73.23280 et FR 80.20861. Les composés de la présente invention sont en effet doués d'une activité analgésique pure de haut niveau. Or, la plupart des substances analgésiques non morphiniques connues à ce jour possèdent également une activité anti-inflammatoire (exemples : salicylés, pyrazolés...) et ils interviennent par conséquent sur les processus intervenant dans l'inflammation. Ceux-ci impliquent de très nombreux médiateurs chimiques (prostaglandines, thomboxane A2...) ; il s'ensuit donc de multiples effets secondaires dont les plus connus sont : attaque de la muqueuse gastrique avec possibilité d'ulcères, inhibition de l'agrégation plaquettaire avec troubles de la coagulation. Outre les dérangements qu'ils occasionnent, ces effets parallèles interdisent l'usage de ces produits chez de nombreux sujets qui y sont particulièrement sensibles. En étant dépourvus de toute activité anti-inflammatoire, les composés de la présente invention n'interviennent pas sur les médiateurs de l'inflammation et sont donc dépourvus des effets secondaires mentionnés précédemment. Cette caractéristique, jointe pour quelques uns d'entre eux à leur absence totale de toxicité et à leur haut niveau d'activité rend certains composés de la présente invention utilisables en tant qu'analgésiques d'une façon beaucoup plus sûre et sans les restrictions d'usage habituellement connues pour la grande majorité de ces produits.

Plus spécifiquement, l'invention concerne les dérivés de formule générale (I) :

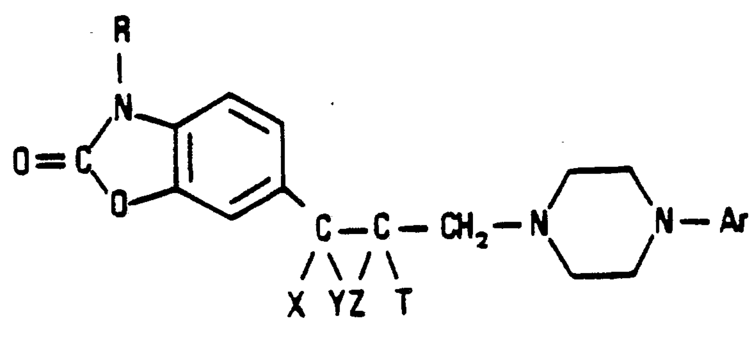

(I)

dans laquelle :

X représente un atome d'hydrogène,

Y représente un atome d'hydrogène ou un groupement hydroxyle ou bien X et Y représentent ensemble un atome d'oxygène,

T représente un atome d'hydrogène ou un groupement alcoyle inférieur,

Z représente un atome d'hydrogène ou bien Z forme avec Y une liaison π, et dans ce cas X représente un atome d'hydrogène,

R représente un atome d'hydrogène ou un groupement alcoyle inférieur,

Ar représente un groupement aryle, ou hétéroaryle, ou alkyl inférieur aryle éventuellement substitué par un atome d'halogène, un groupement alcoyle ou alcoyloxy inférieur, eux- mêmes éventuellement substitués par un ou plusieurs atomes d'halogène,

leurs énantiomères, épimères, diastéréoisomères,

ainsi que leurs sels d'addition à un acide pharmaceutiquement acceptable.

Parmi les acides que l'on peut utiliser pour salifier les composés de formule générale (I), on peut citer, à titre non limitatif les acides chlorhydrique, sulfurique, phosphorique, tartrique, malique, maléique, fumari-

que, oxalique, méthane-sulfonique, éthane-sulfonique, camphorique, citrique etc...

L'invention s'étend aussi à deux procédés d'obtention des composés de formule (I).

Selon les composés de l'invention que l'on souhaite obtenir, il pourra en effet être avantageux d'utiliser soit l'un, soit l'autre procédé.

Le premier procédé de préparation des composés de formule (I), particulièrement intéressant pour l'obtention des composés de formule (I) dans lesquels X, Y, Z représentent chacun un atome d'hydrogène, peut toutefois être appliqué pour les dérivés où X, Y, Z ont d'autres valeurs, utilise comme matière première un dérivé de formule (II) :

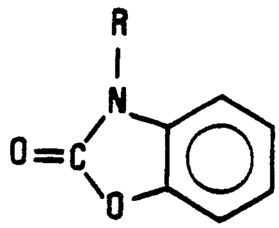

(II)

dans laquelle, R a la même signification que dans la formule (I),
obtenu, par exemple, par réaction de l'orthoaminophénol sur l'urée suivie lorsque R est différent de H d'une alkylation à l'azote,
que l'on soumet à l'action d'un chlorure d'acide de formule (III) :

Cl-CO-HC -$CH_2$-A (III)
T

dans laquelle, T a la même signification que dans la formule (I), A représentant un atome d'halogène,
ou bien de l'anhydride d'acide correspondant,
en présence de chlorure d'aluminium dans le diméthyl formamide selon les conditions de THYES et Coll. (J. Med. Chem. 1983, 26, 6, 800 - 807),
pour obtenir un dérivé de formule (IV) :

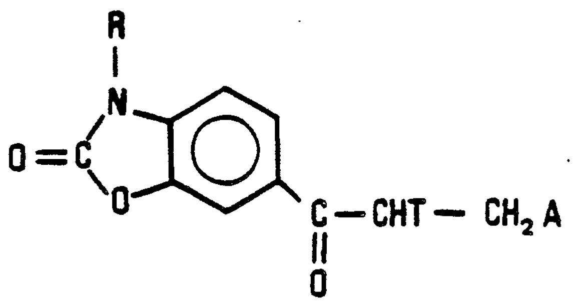

(IV)

dans laquelle R et T ont la même signification que dans la formule (I) et A la même signification que dans la formule (III),
qui, si on le désire, est soumis à réduction par un trialkylsilane en milieu acide selon les conditions décrites par WEST et Coll. (J. Org. Chem. 1973, 38, (15), 2675 - 2681), pour conduire à un dérivé de formule (V) :

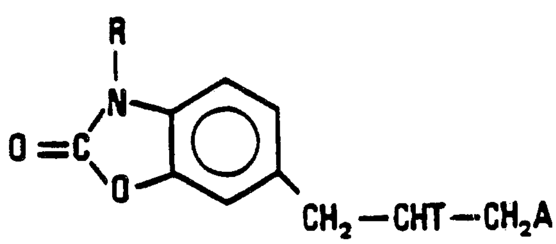

(V)

dans laquelle R et T ont la même signification que dans la formule (I), et A la même signification que dans la formule (III),
le dérivé de formule (IV), ou le dérivé de formule (V), selon la formule du dérivé de formule (I) que l'on

souhaite obtenir, étant alors soumis à l'action d'une aryl - 1 pipérazine de formule (VI) :

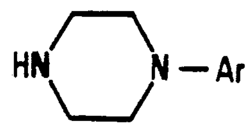

(VI)

dans laquelle Ar a la même signification que dans la formule (I), dans un solvant préférentiellement choisi parmi acétone, acétonitrile, acétate d'éthyle, alcool aliphatique inférieur, dioxanne, benzène, toluène, à une température comprise entre la température ambiante et la température d'ébullition du solvant choisi, en présence d'un excès de l'amine choisie ou d'un capteur de l'hydracide formé tel que la triéthylamine, pour conduire à un dérivé de formule (I/A) :

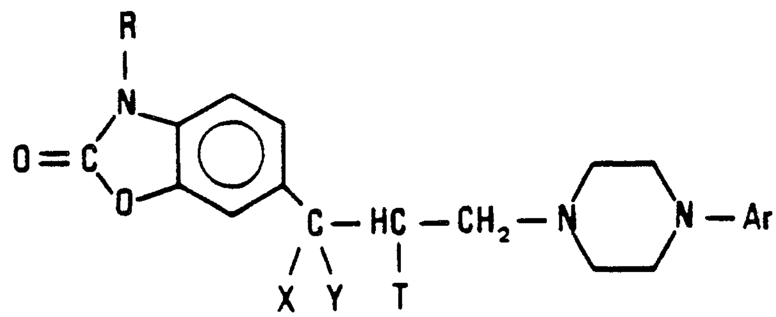

(I/A)

dans laquelle, selon que la matière première utilisée est un dérivé de formule (IV) ou (V),

X et Y représentent ensemble un atome d'oxygène, ou bien X et Y représentent tous deux simultanément un atome d'hydrogène,

R, Ar et T ayant la même signification que dans la formule (I),

qui, si on le désire, est salifié par un acide pharmaceutiquement acceptable ou qui peut, lorsque X et Y représentent ensemble un atome d'oxygène, si on le désire, être soumis,

* ou bien, à un agent d'hydrogénation choisi parmi un hydrure mixte de métal alcalin, comme par exemple le borohydrure de sodium, ou un cyanohydrure mixte de métal alcalin, comme le cyano borohydrure de sodium, préférentiellement en milieu d'alcool aliphatique inférieur, pour conduire à un dérivé de formule (I/B) - majoritairement sous la configuration thréo lorsque T ne représente pas un atome d'hydrogène - :

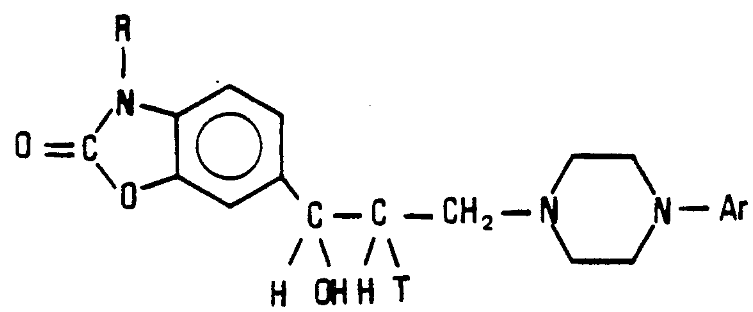

(I/B)

cas particulier des dérivés de formule (I), dans laquelle :

R, T et Ar ont la même signification que dans les dérivés de formule (I),

X représentant ici un atome d'hydrogène,

Y un groupement hydroxyle et Z un atome d'hydrogène,

dont on sépare, si on le désire, les isomères et/ou que l'on salifie par un acide pharmaceutiquement acceptable,

* ou bien à hydrogénation catalytique, sous chauffage et pression dans un solvant choisi parmi alcool aliphatique inférieur ou dioxanne, pour conduire à un dérivé de formule (I/B) - essentiellement sous la configuration érythro lorsque T ne représente pas un atome d'hydrogène - dont on sépare, si on le désire,

les isomères et que l'on salifie le cas échéant par un acide pharmaceutiquement acceptable,
dérivé de formule (I/B) qui, quelque soit le procédé selon lequel il a été obtenu peut être, si on le désire, traité par un agent déshydratant, préférentiellement choisi parmi les hydracides, pour conduire à un dérivé de formule (I/C) :

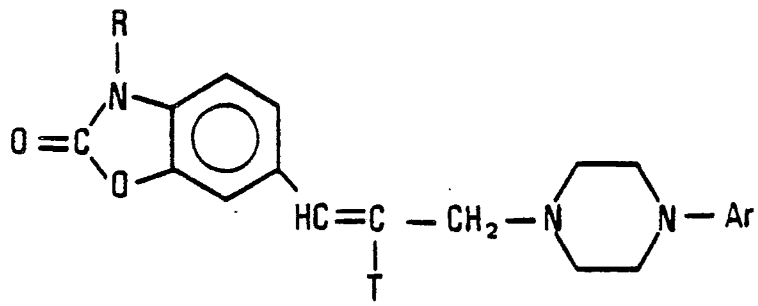

(I/C)

majoritairement sous forme d'isomère trans, cas particulier de dérivés de formule (I), dans laquelle :
R, T et Ar ont la même signification que dans les dérivés de formule (I),
X représentant ici un atome d'hydrogène,
Z formant avec Y une liaison π ,
dont, on sépare, si on le désire, les isomères cis-trans par une technique usuelle telle que la chromatographie sur colonne de silice ou la cristallisation,
et qui, si on le souhaite, peut être salifié par un acide pharmaceutiquement acceptable.

Le second procédé d'obtention des dérivés de la présente invention est inapplicable pour les dérivés pour lesquels R représente un atome d'hydrogène.

Ce second procédé se caractérise en ce que l'on acyle un dérivé de formule (II) obtenu comme indiqué précedemment :

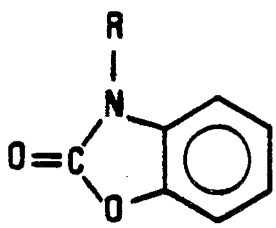

(II)

dans laquelle R représente un groupement alcoyle inférieur par un acide de formule (VII) :

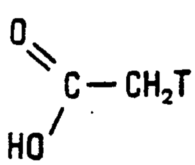

(VII)

dans laquelle T a la même signification que dans la formule (I) ou le chlorure ou l'anhydride de l'acide correspondant selon les conditions décrites dans le brevet FR 73.23280,
pour obtenir un dérivé de formule (VIII) :

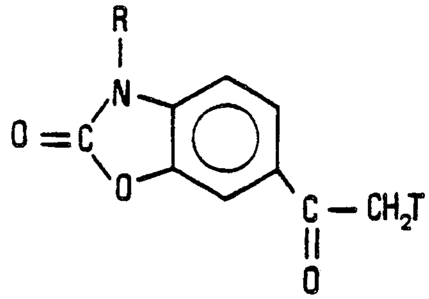

(VIII)

dans laquelle, R représente un groupement alcoyle inférieur, et T a la même signification que dans la formule (I),
que l'on traite ensuite,
* ou bien, selon les conditions de la réaction de Mannich, bien connues de l'homme de l'Art, en présence de trioxyméthylène et de l'aryl pipérazine choisie de formule (VI) :

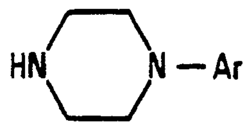

(VI)

dans laquelle, Ar a la même signification que dans la formule (I),
pour obtenir un dérivé de formule (I/A1) :

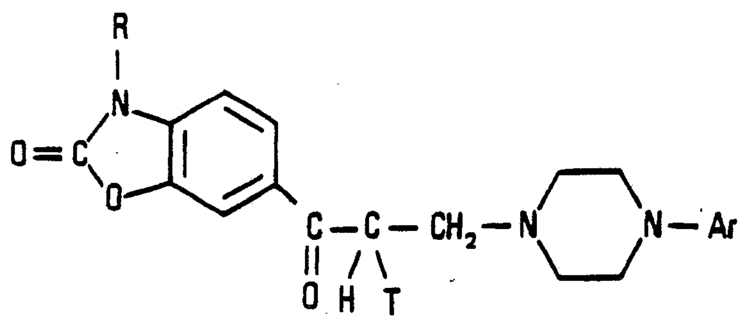

(I/A1)

cas particulier des dérivés de formules (I/A) et (I), dans laquelle :
R représente un groupement alcoyle inférieur, et T et Ar ont la même signification que dans la formule (I),
X et Y représentant ici simultanément un atome d'oxygène et Z un atome d'hydrogène,
* ou bien par le bisdiméthylamino méthane en milieu d'anhydride acétique pour obtenir un produit de formule générale (IX) :

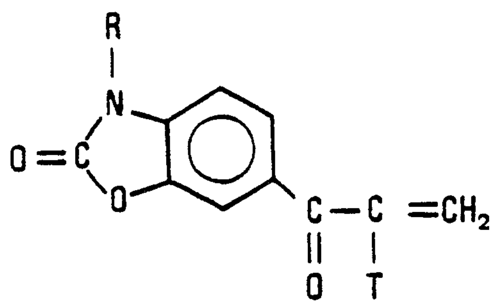

(IX)

dans laquelle :
T a la même signification que dans la formule (I) et R représente un groupement alcoyle inférieur,

que l'on traite par une amine de formule (VI), dans un solvant polaire à une température comprise entre la température ambiante et la température d'ébullition du milieu réactionnel,
pour conduire à un dérivé de formule (I/A1) précédemment défini,
qui, lorsque T ne représente pas un atome d'hydrogène peut être, si on le désire, séparé en ses isomères, que l'on salifie, si on le désire par un acide pharmaceutiquement acceptable et qui,
si on le souhaite, peut être soumis,
* ou bien préférentiellement en milieu d'alcool aliphatique inférieur à un agent d'hydrogénation préférentiellement un hydrure mixte de métal alcalin ou un cyanohydrure mixte de métal alcalin comme par exemple le borohydrure de sodium, ou le cyanoborohydrure de sodium, pour conduire à un dérivé de formule (I/B) majoritairement sous la configuration thréo (lorsque T ne représente pas un atome d'hydrogène) :

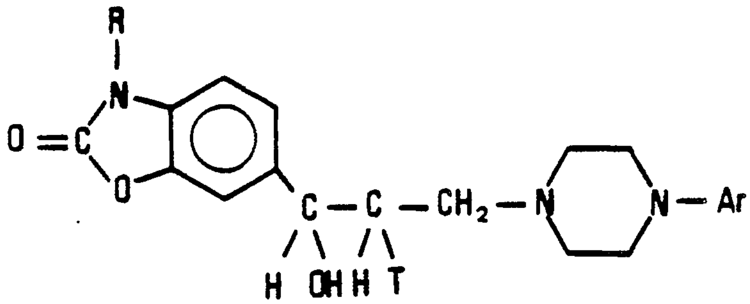

(I/B)

cas particulier des dérivés de formule (I), dans laquelle :
R, T et Ar ont la même signification que dans les dérivés de formule (I),
X représentant ici un atome d'hydrogène,
Y un groupement hydroxyle et Z un atome d'hydrogène,
dont on sépare, si on le désire, les isomères et que l'on peut salifier par un acide pharmaceutiquement acceptable,
* ou bien à hydrogénation catalytique dans un solvant choisi parmi alcool aliphatique inférieur ou dioxanne pour conduire à un dérivé de formule (I/B) essentiellement sous la configuration érythro -lorsque T ne représente pas un atome d'hydrogène - dont on sépare, si on le désire, les isomères et que l'on salifie le cas échéant par un acide pharmaceutiquement acceptable,
dérivé de formule (I/B) que, le cas échéant, on soumet à un agent déshydratant choisi préférentiellement parmi les hydracides pour conduire à un dérivé de formule (I/C), majoritairement sous forme d'isomères trans :

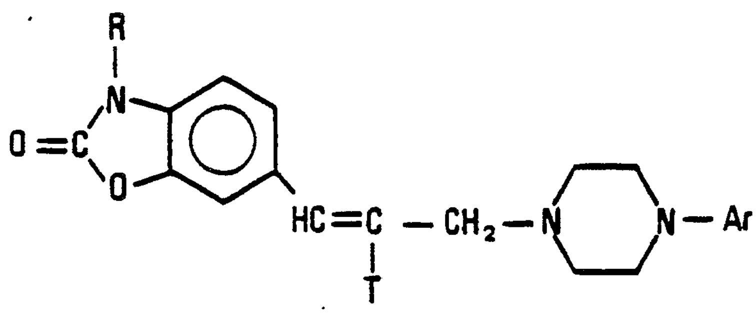

(I/C)

cas particulier de dérivés de formule (I), dans laquelle :
R, T et Ar ont la même signification que dans les dérivés de formule (I),
X représentant ici un atome d'hydrogène,
Z formant avec Y une liaison π ,
dont, si on le désire, on sépare les isomères cis-trans par une technique usuelle telle que la chromatographie sur colonne de silice ou la cristallisation,
et que l'on salifie, si on le désire, par un acide pharmaceutiquement acceptable,
qui, si on le désire, est soumis à une réaction d'hydrogénation catalytique préférentiellement à température et pression ambiantes, et en présence de Nickel de Raney en milieu d'alcool aliphatique inférieur ou de dioxanne pour obtenir un dérivé de formule (I/D) :

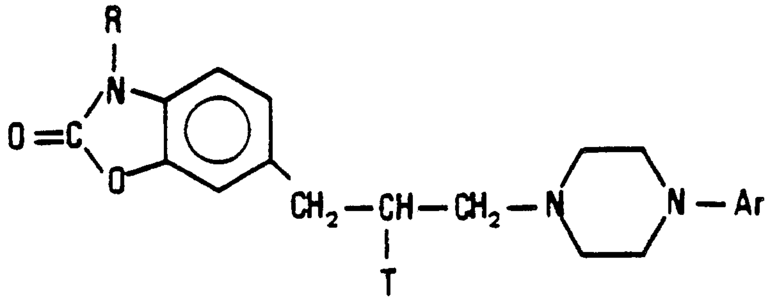

(I/D)

dans laquelle :
R, T et Ar ont la même signification que dans la formule (I),
X, Y et Z représentant chacun simultanément un atome d'hydrogène,
dont on sépare le cas échéant les isomères lorsque T ne représente pas un atome d'hydrogène, et, que l'on salifie éventuellement par un acide pharmaceutiquement acceptable.

Les dérivés de formule (I/D) peuvent également être obtenus à partir des dérivés de formule (IX) :

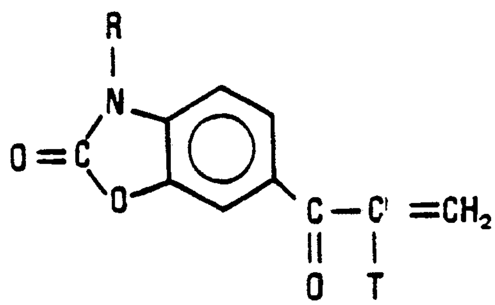

(IX)

dans laquelle :
R et T ont la même signification que dans la formule (I),
que l'on traite par un hydracide pour obtenir un dérivé de formule (IV) :

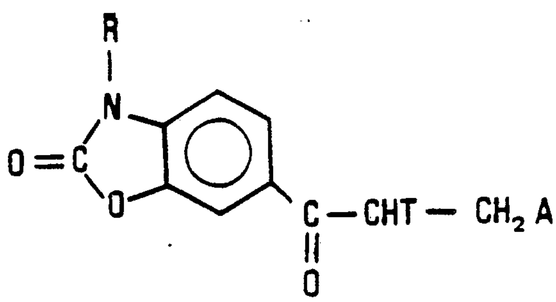

(IV)

dans laquelle R et T ont la même signification que dans la formule (I) et A la même signification que dans la formule (III),
dont on sépare, si on le désire, les isomères lorsque T ne représente pas un atome d'hydrogène,
que l'on soumet à réduction par un trialkylsilane en milieu acide selon les conditions décrites par WEST et Coll, (J. Org. Chem. 1973, <u>38</u>, (15), 2675 - 2681), pour conduire à un dérivé de formule (V) :

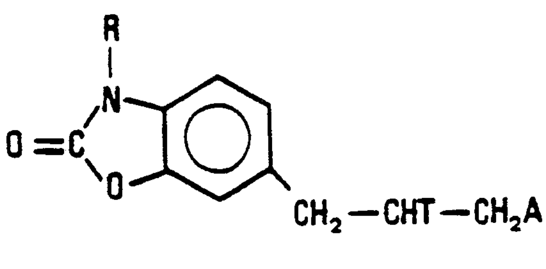

(V)

dans laquelle R et T ont la même signification que dans la formule (I), et A la même signification que dans la formule (III),
que l'on soumet à l'action d'une aryl - 1 pipérazine de formule (VI) :

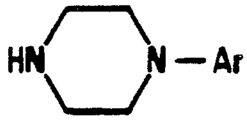

(VI)

dans laquelle Ar a la même signification que dans la formule (I), dans un solvant préférentiellement choisi parmi acétone, acétonitrile, acétate d'éthyle, alcool aliphatique inférieur, dioxanne, benzène, toluène, à une température comprise entre la température ambiante et la température d'ébullition du solvant choisi en présence d'un excès de l'amine choisie ou d'un capteur de l'hydracide formé tel que le triéthylamine,
pour conduire à un dérivé de formule (I/D) ci-dessus référencé, que l'on salifie si on le désire par un acide pharmaceutiquement acceptable.

Les composés de formule (I) possèdent des propriétés pharmacologiques intéressantes.

En particulier ces dérivés ont révélé une activité analgésique intéressante.

L'étude pharmacologique des dérivés de l'invention a en effet montré qu'ils étaient peu toxiques, doués d'une activité analgésique pure et donc dépourvus d'inconvénients inhérents à la plupart des composés non morphiniques présentant cette activité, (action ulcérigène sur les muqueuses, perturbation de la coagulation...). Ce spectre d'activité rend donc les composés de la présente invention intéressants dans un certain nombre d'indications telles que algies rhumatismales, névralgies, lombo-sciatiques, névralgies cervico-brachiales, algies traumatiques telles que entorses, fractures, luxations, douleurs post-traumatiques, douleurs post-opératoires, douleurs dentaires, douleurs neurologiques telles que névralgies faciales, douleurs viscérales telles que coliques néphrétiques, dysménorrhées, chirurgie proctologique, douleurs de la sphère O.R.L., pancréatites, algies diverses, céphalées, douleurs des cancéreux...

La présente invention a également pour objet les compositions pharmaceutiques contenant les produits de formule (I), seuls ou en combinaison avec un ou plusieurs excipients ou véhicules inertes non toxiques, pharmaceutiquement acceptables.

Parmi les compositions pharmaceutiques selon l'invention, on pourra citer plus particulièrement celles qui conviennent pour l'administration orale, parentérale, nasale, les comprimés simples ou dragéifiés, les comprimés sublinguaux, les sachets, les paquets, les gélules, les glossettes, les tablettes, les suppositoires, les crèmes, pommades, gels dermiques, etc...

La posologie utile varie selon l'âge et le poids du patient, la voie d'administration, la nature de l'indication thérapeutique et des traitements éventuellement associées et s'échelonne entre 1 centigramme et 4 grammes par 24 heures.

Les exemples suivants illustrent l'invention et ne la limitent en aucune façon.

Les spectres de résonance magnétique nucléaire $^1H$ ont été enregistrés en utilisant le TMS comme référence interne. Le solvant utilisé est le deutéro chloroforme sauf indication contraire.

Les spectres infra-rouges ont été réalisés à partir d'une pastille de bromure de potassium contenant approximativement 1 % du produit à analyser.

**EXEMPLE 1 : METHYL - 3 {[(TRIFLUOROMETHYL - 3 PHENYL) - 4 PIPERAZINYL - 1] - 3 OXO - 1 PROPYL} - 6 BENZOXAZOLINONE**

Dans une fiole rodée de 250 $cm^3$, munie d'un réfrigérant à reflux, dissoudre sous agitation magnétique 0,03 mole d'acétyl - 6 méthyl - 3 benzoxazolinone obtenue comme décrit dans le brevet Fr 73.23280 et 0,045 mole du chlorhydrate de (trifluorométhyl - 3 phényl) - 1 pipérazine dans 150 $cm^3$ d'éthanol. Ajouter 0,045 mole de trioxyméthylène et acidifier par l'acide chlorhydrique, chauffer à reflux pendant 72 heures. Essorer le précipité formé, laver à l'acétone, placer en suspension dans l'eau, alcaliniser par la soude. Extraire à plusieurs reprises au chloroforme, sécher les phases organiques sur chlorure de calcium, filtrer et évaporer au bain-marie sous vide. Recristalliser le résidu dans l'éthanol.

Rendement : 70 %

Point de fusion : 137 °C

Caractéristiques spectrales :

Infra-rouge :

1770 $cm^{-1}$ : ν CO (O - CO - N)
1665 $cm^{-1}$ : ν CO (acyle)
Résonance magnétique nucléaire :
δ = 2,70 ppm, massif, 6H, $CH_2$ - N et pipéraziniques
δ = 3,20 ppm, massif, 6H, CO - $CH_2$ et pipéraziniques
δ = 3,45 ppm, singulet, 3H, $CH_3$ ; N - $CH_3$

**EXEMPLE 2 : METHYL - 3 {[(METHOXY - 2 PHENYL) - 4 PIPERAZINYL - 1] -3 OXO - 1 PROPYL} - 6 BENZOXAZOLINONE**

En procédant comme dans l'exemple 1, mais en remplaçant la (trifluorométhyl - 3 phényl) - 1 pipérazine par la (méthoxy - 2 phényl) -1 pipérazine, on obtient le produit du titre.
Rendement : 70 %
Point de fusion : 152 °C
Caractéristiques spectrales :
Infra-rouge :
1760 $cm^{-1}$ : ν CO (O - CO - N)
1670 $cm^{-1}$ : ν CO (acyle)
Résonance magnétique nucléaire (Solvant $CDCl_3$) :
δ = 2,80 ppm, massif, 6H, $CH_2$ - N et pipéraziniques
δ = 3,15 ppm, massif, 6H, CO - $CH_2$ et pipéraziniques
δ = 3,50 ppm, singulet, 3H, N - $CH_3$
δ = 3,90 ppm, singulet, 3H, $OCH_3$

**EXEMPLE 3 : METHYL - 3 {[(FLUORO - 4 PHENYL) - 4 PIPERAZINYL - 1] -3 OXO - 1 PROPYL} - 6 BENZOXAZOLINONE**

En procédant comme dans l'exemple 1, mais en remplaçant la (trifluorométhyl - 3 phényl) - 1 pipérazine, par la (fluoro - 4 phényl) -1 pipérazine, on obtient le produit attendu.
Rendement : 70 %
Point de fusion : 149 °C
Caractéristiques spectrales :
Infra-rouge :
1770 $cm^{-1}$ : ν CO (O - CO - N)
1665 $cm^{-1}$ : ν CO (acyle)
Résonance magnétique nucléaire (Solvant $CDCl_3$) :
δ = 2,75 ppm, massif, 6H, $CH_2$ - N et pipéraziniques
δ = 3,15 ppm, massif, 6H, CO - $CH_2$ et pipéraziniques
δ = 3,45 ppm, singulet, 3H, N - $CH_3$

**EXEMPLE 4 : METHYL - 3 {[(PYRIMIDINYL -2) - 4 PIPERAZINYL - 1] - 3 OXO - 1 PROPYL} - 6 BENZOXAZOLINONE**

En procédant comme dans l'exemple 1, mais en remplaçant la (trifluorométhyl - 3 phényl) - 1 pipérazine, par la (pyrimidinyl - 2) - 1 pipérazine, on obtient le produit du titre.

**EXEMPLE 5 : METHYL - 3 {[(TRIFLUOROMETHYL - 3 PHENYL) - 4 PIPERAZINYL - 1] - 3 HYDROXY - 1 PROPYL} - 6 BENZOXAZOLINONE**

**STADE A** : Méthyl - 3 {[(trifluorométhyl - 3 phényl) - 4 pipérazinyl -1] - 3 oxo - 1 propyl} - 6 benzoxazolinone

Voir exemple 1.

**STADE B** : Méthyl - 3 {[(trifluorométhyl - 3 phényl) - 4 pipérazinyl - 1] - 3 hydroxy - 1 propyl} - 6 benzoxazolinone

Dans une fiole de 250 $cm^3$ munie d'une agitation magnétique, dissoudre 0,01 mole de méthyl - 3 {[-(trifluorométhyl - 3 phényl) - 4 pipérazinyl - 1] - 3 oxo - 1 propyl} - 6 benzoxazolinone dans 200 $cm^3$ de méthanol. Ajouter très lentement et sous agitation 0,02 mole de borohydrure de sodium. Maintenir l'agitation 4 heures à température ambiante. Evaporer le milieu réactionnel au bain-marie sous vide. Reprendre le résidu par l'eau et extraire plusieurs fois au chloroforme. Filtrer et évaporer à sec au bain-marie sous vide. Recristalliser dans l'éthanol.
Rendement : 75 %
Point de fusion : 139 °C
Caractéristiques spectrales :
Infra-rouge :
3240 $cm^{-1}$ : ν OH (alcool secondaire)
1750 $cm^{-1}$ : ν CO (O - CO - N)
Résonance magnétique nucléaire :
δ = 1,83 ppm, massif, 2H, CHOH - $CH_2$ - $CH_2$
δ = 2,75 ppm, massif, 6H, $CH_2$ - N et pipéraziniques
δ = 3,35 ppm, massif, 7H, N - $CH_3$ et pipéraziniques
δ = 4,95 ppm, triplet, 1H, CHOH, J = 5,6Hz

**EXEMPLE 6 : METHYL - 3 {[(METHOXY - 2 PHENYL) - 4 PIPERAZINYL - 1] -3 HYDROXY - 1 PROPYL} - 6 BENZOXAZOLINONE**

En procédant comme dans l'exemple 5, mais en remplaçant la méthyl -3 {[(trifluorométhyl - 3 phényl) - 4 pipérazinyl - 1] - 3 oxo - 1 propyl} - 6 benzoxazolinone par la méthyl - 3 {[(méthoxy - 2 phényl) - 4 pipérazinyl - 1] - 3 oxo - 1 propyl} - 6 benzoxazolinone, on obtient le produit du titre.
Point de fusion : 133 - 134 °C
Caractéristiques spectrales :
Infra-rouge :
3140 $cm^{-1}$ : ν OH (alcool secondaire)
1770 $cm^{-1}$ : ν CO (O - CO - N)
Résonance magnétique nucléaire :
δ = 1,88 ppm, massif, 2H, CHOH - $CH_2$ - $CH_2$
δ = 2,80 ppm, massif, 6H, $CH_2$ - N et pipéraziniques
δ = 3,15 ppm, triplet, 4H, pipéraziniques, J = 4,7Hz
δ = 3,38 ppm, singulet, 3H, N $CH_3$
δ = 3,85 ppm, singulet, 3H, $OCH_3$
δ = 4,96 ppm, triplet, 1H, CH - OH

**EXEMPLE 7 : METHYL - 3 {[(FLUORO - 4 PHENYL) - 4 PIPERAZINYL - 1] -3 HYDROXY - 1 PROPYL} - 6 BENZOXAZOLINONE**

En procédant comme dans l'exemple 5, mais en remplaçant la méthyl -3 {[(trifluorométhyl - 3 phényl) - 4 pipérazinyl - 1] - 3 oxo - 1 propyl}, - 6 benzoxazolinone par la méthyl - 3 {[(fluoro - 4 phényl) - 4 pipérazinyl - 1] - 3 oxo - 1 propyl} - 6 benzoxazolinone, on obtient le produit du titre.
Point de fusion : 150 - 151 °C
Caractéristiques spectrales :
Infra-rouge :
3450 $cm^{-1}$ : ν OH(alcool secondaire)
1745 $cm^{-1}$ : ν CO (O - CO - N)
Résonance magnétique nucléaire :
δ = 1,83 ppm, massif, 2H, $CH_2$ - $CH_2$ - N
δ = 2,70 ppm, massif, 6H, $CH_2$ - N et pipéraziniques

δ = 3,18 ppm, triplet, 4H, pipéraziniques, J = 4,6Hz
δ = 3,37 ppm, singulet, 3H, N - $CH_3$
δ = 4,96 ppm, triplet, 1H, CH - OH

**EXEMPLE 8 : METHYL - 3 {[(TRIFLUOROMETHYL - 3 PHENYL) - 4 PIPERAZINYL - 1] - 3 PROPEN - 1 YL} - 6 BENZOXAZOLINONE**

**STADE A** : Méthyl - 3 {[(trifluorométhyl - 3 phényl) - 4 pipérazinyl - 1] - 3 hydroxy - 1 propyl} - 6 benzoxazolinone

Obtenue dans l'exemple 5.

**STADE B** : Méthyl - 3 {[(trifluorométhyl - 3 phényl) - 4 pipérazinyl - 1] - 3 propen - 1 yl} - 6 benzoxazolinone

Dans une fiole de 250 $cm^3$ dissoudre 0,015 mole de méthyl - 3 {[(trifluorométhyl - 3 phényl) - 4 pipérazinyl -1] - 3 hydroxy - 1 propyl} - 6 benzoxazolinone, dans l'acide bromhydrique à 47 %, et agiter la solution à la température ambiante pendant 2 heures. Essorer le précipité obtenu, le laver à l'acétone, le placer en suspension dans l'eau, alcaliniser par la soude. Extraire à plusieurs reprises au chloroforme, réunir et sécher les phases organiques sur chlorure de calcium, filtrer, évaporer à sec au bain-marie sous vide et recristalliser la base dans le cyclohexane.
Rendement : 80 %
Point de fusion : 97 °C
Caractéristiques spectrales :
Infra-rouge : 1765 $cm^{-1}$ : ν CO (O - CO - N)
Résonance magnétique nucléaire :
δ = 3,35 ppm, singulet, 3H, (N $CH_3$)
δ = 6,20 ppm, triplet dédoublé, 1H, - CH - $CH_2$
δ = 6,62 ppm, doublet, 1H, - CH = CH, J = 15,9 Hz

**EXEMPLE 9 : METHYL - 3 {[(METHOXY - 2 PHENYL) - 4 PIPERAZINYL - 1] -3 PROPEN - 1 YL} - 6 BENZOXAZOLINONE**

En procédant comme dans l'exemple 8, mais en remplaçant la méthyl -3 {[(trifluorométhyl - 3 phényl) - 4 pipérazinyl - 1] - 3 hydroxy - 1 propyl} - 6 benzoxazolinone, par la méthyl - 3 {[(méthoxy - 2 phényl) - 4 pipérazinyl - 1] - 3 hydroxy - 1 propyl} - 6 benzoxazolinone obtenue dans l'exemple 6, on obtient le produit du titre.
Solvant de recristallisation : éthanol
Rendement : 80 %
Point de fusion : 151 °C
Caractéristiques spectrales :
Infra-rouge : 1760 $cm^{-1}$ : ν CO (O - CO - N)
Résonance magnétique nucléaire :
δ = 3,38 ppm, singulet, 3H, N - $CH_3$
δ = 3,83 ppm, singulet, 3H, - $OCH_3$
δ = 6,19 ppm, triplet dédoublé, 1H = CH - $CH_2$
δ = 6,60 ppm, doublet, 1H, CH = CH, J = 16 Hz

**EXEMPLE 10 : METHYL - 3 {[(FLUORO - 4 PHENYL) - 4 PIPERAZINYL - 1] - 3 PROPEN - 1 YL} - 6 BENZOXAZOLINONE**

En procédant comme dans l'exemple 8, mais en remplaçant la méthyl -3 {[(trifluorométhyl - 3 phényl) - 4 pipérazinyl - 1] - 3 hydroxy - 1 propyl} - 6 benzoxazolinone, par la méthyl - 3 {[(fluoro - 4 phényl) - 4 pipérazinyl - 1] - 3 hydroxy - 1 propyl} - 6 benzoxazolinone obtenue dans l'exemple 7, on obtient le produit

du titre.
Point de fusion : 164 °C
Caractéristiques spectrales :
Infra-rouge : 1770 $cm^{-1}$ : ν CO (O - CO - N)
Résonance magnétique nucléaire :
δ = 3,38 ppm, singulet, 3H, N - $CH_3$
δ = 6,20 ppm, triplet dédoublé, 1H, = CH - $CH_2$, J = 16,1 Hz
δ = 6,55 ppm, doublet, 1H, - CH, J = 16, 1 Hz

**EXEMPLE 11: METHYL - 3 {[(TRIFLUOROMETHYL - 3 PHENYL) - 4 PIPERAZINYL - 1] - 3 PROPYL} - 6 BENZOXAZOLINONE (CHLORHYDRATE)**

**STADE A** : Méthyl - 3 {[(trifluorométhyl - 3 phényl) - 4 pipérazinyl - 1] - 3 propen - 1 yl} - 6 benzoxazolinone

Obtenue dans l'exemple 8.

**STADE B** : Méthyl - 3 {[(trifluorométhyl - 3 phényl) - 4 pipérazinyl - 1] - 3 propyl} - 6 benzoxazolinone

Dans une fiole conique de 500 $cm^3$ munie d'un robinet 3 voies et d'une agitation magnétique, dissoudre 0,01 mole de méthyl - 3 {[(trifluorométhyl - 3 phényl) - 4 pipérazinyl - 1] - 3 propen - 1 yl} - 6 benzoxazolinone, dans le méthanol puis ajouter 0,5 g de nickel de Raney. Agiter sous atmosphère d'hydrogène à température et pressions ordinaires. Après absorption de la quantité théorique d'hydrogène, filtrer le milieu réactionnel, évaporer à sec le filtrat au bain-marie sous vide. Reprendre le résidu par l'eau, acidifier par l'acide chlorhydrique, essorer le précipité obtenu et recristalliser dans l'éthanol.
Point de fusion : 234 °C
Caractéristiques spectrales :
Infra-rouge :
2540 - 2420$cm^{-1}$ : ν $NH^+$ (chlorhydrate d'amine tertiaire)
1780 - 1760 $cm^{-1}$ : ν CO (O - CO - N)
Résonance magnétique nucléaire :
δ = 1,90 ppm, quintuplet, 2H, CH2 - $CH_2$ - $CH_2$
δ = 2,67 ppm, massif, 8H, $CH_2$ - $CH_2$ - $CH_2$ et pipéraziniques
δ = 3,34 ppm, massif, 7H, N - $CH_3$ et pipéraziniques.

**EXEMPLE 12 : METHYL - 3 {[(METHOXY - 2 PHENYL) - 4 PIPERAZINYL -1] - 3 PROPYL} - 6 BENZOXAZOLINONE (BROMHYDRATE)**

**STADE A :** Méthyl - 3 {[(méthoxy - 2 phényl) - 4 pipérazinyl - 1] -3 propen - 1 yl} - 6 benzoxazolinone

Obtenue dans l'exemple 9.

**STADE B :** Méthyl - 3 {[(méthoxy - 2 phényl) - 4 pipérazinyl - 1] -3 propyl} - 6 benzoxazolinone

Dans une fiole de 500 $cm^3$ munie d'un robinet 3 voies et d'une agitation magnétique, dissoudre 0,01 mole de méthyl - 3 {[(méthoxy - 2 phényl) - 4 pipérazinyl - 1] - 3 propen - 1 yl} - 6 benzoxazolinone dans l'éthanol, puis ajouter 0,5 g de nickel de Raney. Agiter sous atmosphère d'hydrogène à température et pression ordinaires. Après absorption de la quantité théorique d'hydrogène, filtrer le milieu réactionnel et évaporer à sec le filtrat au bain-marie sous vide. Reprendre le résidu par l'alcool absolu, faire barboter un courant d'acide bromhydrique gazeux jusqu'à précipitation du bromhydrate d'amine, essorer et recristalliser dans l'alcool à 95 %.
Point de fusion : 222 °C
Caractéristiques spectrales :
Infra-rouge :

2660 - 2540 $cm^{-1}$ : ν $NH^+$ (amine tertiaire)
1765 $cm^{-1}$ : ν CO (O - CO - N)
Résonance magnétique nucléaire :
δ = 2,40 ppm, quintuplet, 2H, $CH_2$ - $CH_2$ - $CH_2$
δ = 3,00 ppm, massif, 6H, $CH_2$ - N et pipéraziniques
δ = 3,37 ppm, singulet, 3H, N - $CH_3$
δ = 3,55 ppm, massif, 6H, $CH_2$ - $CH_2$ - $CH_2$ et pipéraziniques
δ = 3,83 ppm, singulet, 3H, $OCH_3$

**EXEMPLE 13 : METHYL - 3 {[(FLUORO - 4 PHENYL) - 4 PIPERAZINYL - 1] - 3 PROPYL} - 6 BENZOXAZOLINONE**

**STADE A :** Méthyl - 3 {[(fluoro - 4 phényl) - 4 pipérazinyl - 1] -3 propen - 1 yl} - 6 benzoxazolinone

Obtenue dans l'exemple 10.

**STADE B :** Méthyl - 3 {[(fluoro - 4 phényl) - 4 pipérazinyl - 1] -3 propyl} - 6 benzoxazolinone

Dans une fiole de 500 $cm^3$ munie d'un robinet 3 voies et d'une agitation magnétique, dissoudre 0,01 mole de méthyl - 3 {[(fluoro - 4 phényl) - 4 pipérazinyl - 1] - 3 propen - 1 yl} - 6 benzoxazolinone dans l'acétate d'éthyle puis ajouter 0,5 g de nickel de Raney. Agiter, sous atmosphère d'hydrogène à température et pression ordinaires. Après absorption de la quantité théorique d'hydrogène, filtrer le milieu réactionnel et évaporer à sec au bain-marie sous vide. Le résidu est cristallisé dans l'éthanol.
Point de fusion : 108 °C
Caractéristiques spectrales :
Infra-rouge : 1765 $cm^{-1}$ : ν CO (O - CO - N)
Résonance magnétique nucléaire :
δ = 1,88 ppm, quintuplet, 2H, $CH_2$ - $CH_2$ - $CH_2$
δ = 2,59 ppm, massif, 8H, $CH_2$ - $CH_2$ - $CH_2$ et pipéraziniques
δ = 3,12 ppm, triplet, 4H, pipéraziniques
δ = 3,40 ppm, singulet, 3H, N - $CH_3$

**EXEMPLE 14 : METHYL - 3 {[(TRIFLUOROMETHYL - 3 PHENYL) - 4 PIPERAZINYL - 1] - 3 METHYL - 2 PROPIONYL} - 6 BENZOXAZOLINONE**

Dans une fiole à col rodé de 250 $cm^3$ munie d'un réfrigérant à reflux et d'une agitation magnétique, dissoudre 0,025 mole de propionyl - 6 méthyl - 3 benzoxazolinone décrite dans le brevet Fr. 73.23280, et 0,038 mole de chlorhydrate de (trifluorométhyl - 3 phényl) - 1 pipérazine dans 150 $cm^3$ de propanol. Ajouter 0,038 mole de trioxyméthylène et 1,5 $cm^3$ d'acide chlorhydrique concentré. Chauffer à reflux pendant 72 heures. Essorer le précipité formé, le laver à l'acétone, le placer en suspension dans l'eau et alcaliniser par la soude. Extraire plusieurs fois au chloroforme, réunir les phases organiques, sécher sur chlorure de calcium, filtrer, évaporer au bain-marie sous vide et recristalliser le résidu dans le propanol
Rendement : 74 %
Point de fusion : 92 °C
Caractéristiques spectrales :
Infra-rouge :
1780 $cm^{-1}$ : ν CO (O - CO - N)
1670 $cm^{-1}$ : ν CO (acyl)
Résonance magnétique nucléaire :
δ = 1,23 ppm, doublet, 3H, CH - $CH_3$, J = 5,8 Hz
δ = 2,72 ppm, massif, 6H, CH - $CH_2$ et pipéraziniques
δ = 3,17 ppm, triplet, 4H, pipéraziniques
δ = 3,41 ppm, singulet, 3H, N - $CH_3$
δ = 3,75 ppm, massif, 1H, CH - $CH_3$

**EXEMPLE 15 : METHYL - 3 {[(METHOXY - 2 PHENYL) - 4 PIPERAZINYL -1] - 3 METHYL - 2 PROPIONYL} - 6 BENZOXAZOLINONE**

**STADE A :** Méthyl - 3 (méthylène - 2 propionyl) - 6 benzoxazolinone

Dans une fiole à col rodé de 150 $cm^3$ munie d'un réfrigérant à reflux et d'une agitation magnétique introduire 0,01 mole de méthyl - 3 propionyl - 6 benzoxazolinone et 0,04 mole de bis (diméthylamino) méthane. Ajouter goutte à goutte 15 $cm^3$ d'anhydride acétique et chauffer à 100 °C pendant 5 heures. Après refroidissement, verser le mélange réactionnel dans 5 volumes d'eau glacée. Acidifier et agiter une heure, essorer, laver à l'eau jusqu'à neutralité, sécher et recristalliser dans l'hexane.
Rendement : 66 %
Point de fusion : 82 °C

**STADE B :** Méthyl - 3 {[(méthoxy - 2 phényl) - 4 pipérazinyl - 1] -3 méthyl - 2 propionyl} - 6 benzoxazolinone

Dans une fiole rodée de 250 $cm^3$ munie d'un réfrigérant à reflux et d'une agitation magnétique, introduire successivement 100 $cm^3$ d'acétone anhydre, 0,01 mole de méthyl - 3 (méthylène - 2 propionyl) - 6 benzoxazolinone et 0,01 mole de (méthoxy - 2 phényl) - 1 pipérazine. Porter 6 heures à reflux, laisser refroidir. Essorer : on obtient le produit du titre.
Rendement : 82%
Point de fusion : 156 °C
Caractéristiques spectrales :
Infra-rouge :
1785 $cm^{-1}$ : ν CO (O - CO - N)
1660 $cm^{-1}$ : ν CO (acyl)
Résonance magnétique nucléaire :
δ = 1,24 ppm, doublet, 3H, CH - $CH_3$
δ = 2,60 ppm, massif, 6H, CH - $CH_2$ et pipéraziniques
δ = 3,00 ppm, triplet, 4H, pipéraziniques
δ = 3,39 ppm, singulet, 3H, N - $CH_3$
δ = 3,63 ppm, massif, 1H, CH - $CH_3$
δ = 3,84 ppm, singulet, 3H, $OCH_3$

**EXEMPLE 16 : (±) ERYTHRO METHYL - 3{[(TRIFLUOROMETHYL - 3 PHENYL) - 4 PIPERAZINYL - 1] - 3 HYDROXY - 1 METHYL -2 PROPYL} - 6 BENZOXAZOLINONE**

Dans un autoclave de 250 $cm^3$, dissoudre 0,01 mole de méthyl - 3 {[(trifluorométhyl - 3 phényl) - 4 pipérazinyl - 1] - 3 méthyl - 2 propionyl} - 6 benzoxazolinone, obtenue dans l'exemple 14, dans l'éthanol et ajouter 0,5 g de charbon palladié à 10 %. Hydrogéner sous 50 atmosphères d'hydrogène pendant 8 heures à une température voisine de 70 °C. Après refroidissement, éliminer l'hydrogène, filtrer et évaporer le filtrat au bain-marie sous vide. Recristalliser le résidu dans l'éthanol dilué.
Rendement : 67 %
Point de fusion : 120 °C
Caractéristiques spectrales :
Infra-rouge :
3210 $cm^{-1}$ : ν OH (alcool)
1765 $cm^{-1}$ : ν CO ( O - CO - N)
Résonance magnétique nucléaire :
δ = 0,8 ppm, doublet, 3H, CH - $CH_3$, J = 5,7 Hz
δ = 4,89 ppm, doublet, 1H, CH - OH, J = érythro = 2,5 Hz

**EXEMPLE 17 : (±) ERYTHRO METHYL - 3 {[(METHOXY - 2 PHENYL) - 4 PIPERAZINYL - 1] - 3 HYDROXY - 1 METHYL - 2 PROPYL} - 6 BENZOXAZOLINONE**

En procédant comme dans l'exemple 16, mais en hydrogénant la méthyl - 3 {[(méthoxy - 2 phényl) - 4 pipérazinyl - 1] - 3 méthyl - 2 propionyl} - 6 benzoxazolinone obtenue dans l'exemple 15 (solvant d'hydrogénation : dioxanne), on obtient le produit du titre.

Rendement : 70 %
Point de fusion : 114 °C
Caractéristiques spectrales :
Infra-rouge :
3185 $cm^{-1}$ : ν OH (alcool secondaire)
1765 $cm^{-1}$ : ν CO (O - CO - N)
Résonance magnétique nucléaire :
δ = 0,77 ppm, doublet, 3H, CH - $CH_3$, J = 5,6 Hz
δ = 3,40 ppm, singulet, 3H, N - $CH_3$
δ = 3,87 ppm, singulet, 3H, O - $CH_3$
δ = 4,86 ppm, doublet, 1H, CH - OH, J érythro = 2,7 Hz

**EXEMPLE 18 : (±) THREO METHYL - 3{[(TRIFLUOROMETHYL - 3 PHENYL) -4 PIPERAZINYL - 1] - 3 HYDROXY - 1 METHYL - 2 PROPYL} - 6 BENZOXAZOLINONE**

Placer 0,01 mole de méthyl - 3 {[(trifluorométhyl - 3 phényl) - 4 pipérazinyl - 1] - 3 méthyl - 2 propionyl} - 6 benzoxazolinone obtenue dans l'exemple 14 dans 25 $cm^3$ de méthanol. Ajouter par petites fractions et sous agitation 0,02 mole de borohydrure de sodium. Maintenir l'agitation deux heures à température ambiante. Evaporer le solvant au bain-marie sous vide. Reprendre le résidu par l'eau et extraire au chloroforme. Sécher la phase organique sur chlorure de calcium, filtrer et évaporer au bain-marie sous vide. On obtient un mélange d'énantiomères à partir duquel le dérivé thréo majoritaire est isolé par chromatographie sur gel de silice (éluant chlorure de méthylène, acétone : 8,5/1,5).

Rendement : 30 %
Point de fusion : 80 °C
Caractéristiques spectrales :
Infra-rouge :
3110 $cm^{-1}$ : ν OH (alcool secondaire)
1770 $cm^{-1}$ : ν CO (O - CO - N) benzoxazolinone
Résonance magnétique nucléaire :
δ = 0,58 ppm, doublet, 3H, CH - $CH_3$, J = 5,8 Hz
δ = 4,40 ppm, doublet, 1H, CH - OH, J érythro = 8,8 Hz

**EXEMPLE 19 : (±) THREO METHYL - 3 {[(METHOXY - 2 PHENYL) - 4 PIPERAZINYL - 1] - 3 HYDROXY - 1 METHYL - 2 PROPYL} -6 BENZOXAZOLINONE**

En procédant comme dans l'exemple 18, mais en utilisant comme matière première la méthyl - 3 {[(méthoxy - 2 phényl) - 4 pipérazinyl - 1] - 3 méthyl - 2 propionyl} - 6 benzoxazolinone obtenue dans l'exemple 15, on obtient le produit du titre. Solvant d'élution : chloroforme, acétone : 4/1.

Rendement : 32 %
Point de fusion : 64 °C
Caractéristiques spectrales :
Infra-rouge :
3210 $cm^{-1}$ : ν OH (alcool secondaire)
1765 $cm^{-1}$ : ν CO (O - CO - N) benzoxazolinone
Résonance magnétique nucléaire :
δ = 0,58 ppm, doublet, 3H, CH - $CH_3$, J = 6 Hz
δ = 3,38 ppm, singulet, 3H, N - $CH_3$
δ = 3,91 ppm, singulet, 3H, O - $CH_3$
δ = 4,44 ppm, doublet, 1H, CH - OH, J = thréo = 8,95 Hz

**EXEMPLE 20 : METHYL - 3{[(TRIFLUOROMETHYL - 3 PHENYL) - 4 PIPERAZINYL -1] - 3 METHYL - 2 PROPEN - 1 - YL} - 6 BENZOXAZOLINONE**

Dans une fiole de 250 $cm^3$ munie d'une agitation magnétique, dissoudre 0,01 mole de méthyl - 3 {[-(trifluorométhyl - 3 phényl) - 4 pipérazinyl - 1] - 3 méthyl - 2 hydroxy - 1 propyl] - 6 benzoxazolinone, obtenue dans l'exemple 16, dans 100 $cm^3$ d'acide bromhydrique à 47 %. Chauffer au bain-marie à 40 °C et poursuivre l'agitation magnétique pendant 2 heures. Essorer le précipité formé, laver à l'acétone, mettre en suspension dans l'eau et alcaliniser. Extraire au chloroforme, sécher les phases organiques sur chlorure de calcium, filtrer, évaporer à sec au bain-marie sous vide et recristalliser dans l'hexane.

Rendement : 71 %
Point de fusion : 92 °C
Caractéristiques spectrales :
Infra-rouge : 1790 - 1779 $cm^{-1}$ : ν CO (O - CO - N)
Résonance magnétique nucléaire:
δ = 1,95 ppm, singulet, 3H, CH - $CH_3$
δ = 3,40 ppm, singulet, 3H, N - $CH_3$
δ = 6,45 ppm, singulet, 1H, - CH =

**EXEMPLE 21 : METHYL - 3 {[(METHOXY - 2 PHENYL) - 4 PIPERAZINYL -1] - 3 METHYL - 2 PROPEN - 1 - YL} - 6 BENZOXAZOLINONE**

En procédant comme dans l'exemple 20, mais en utilisant comme matière première la méthyl - 3 {[-(méthoxy - 2 phényl) - 4 pipérazinyl - 1] - 3 méthyl - 2 hydroxy - 1 propyl} - 6 benzoxazolinone, on obtient le produit du titre.

Solvant de recristallisation : éthanol
Rendement : 82 %
Point de fusion : 148 °C
Caractéristiques spectrales :
Infra-rouge : 1780 $cm^{-1}$ : ν CO (O - CO - N)
Résonance magnétique nucléaire
δ = 1,90 ppm, singulet, 3H, C - $CH_3$
δ = 3,78 ppm, singulet, 3H, N - $CH_3$
δ = 3,85 ppm, singulet, 3H, O - $CH_3$
δ = 6,50 ppm, singulet, 1H, - CH =

**EXEMPLE 22 : METHYL - 3 {[(TRIFLUOROMETHYL - 3 PHENYL) - 4 PIPERAZINYL - 1] - 3 METHYL - 2 PROPYL} - 6 BENZOXAZOLINONE (CHLORHYDRATE)**

Dans une fiole de 500 $cm^3$ munie d'un robinet à trois voies et d'une agitation magnétique, dissoudre 0,01 mole de méthyl - 3 {[(trifluorométhyl - 3 phényl) - 4 pipérazinyl - 1] - 3 méthyl - 2 propen - 1 yl} - 6 benzoxazolinone obtenue dans l'exemple 20, dans l'éthanol absolu. Ajouter 0,5 g de nickel de Raney et agiter sous atmosphère d'hydrogène à température et pression ordinaires. Après absorption de la quantité théorique d'hydrogène, filtrer le milieu réactionnel, évaporer à sec le filtrat au bain-marie sous vide. Reprendre le résidu par l'eau, acidifier, essorer et recristalliser dans l'éthanol.

Rendement : 80 %
Point de fusion : 261 °C
Caractéristiques spectrales :
Infra-rouge :
2520 - 2490 $cm^{-1}$ : ν $NH^+$ ($Cl^-$ d'amine tertiaire)
1765 $cm^{-1}$ : ν (CO) (O - CO - N)
Résonance magnétique nucléaire (solvant DMSO) :
δ = 0,94 ppm, doublet, 3H, $CH_3$ - CH, J = 6,1 Hz
δ = 3,37 ppm, singulet, 3H, N - $CH_3$

**EXEMPLE 23 : METHYL - 3 {[(METHOXY - 2 PHENYL) - 4 PIPERAZINYL -1] - 3 METHYL - 2 PROPYL} - 6 BENZOXAZOLINONE**

Dans une fiole de 500 $cm^3$ munie d'un robinet à trois voies et d'une agitation magnétique, dissoudre

0,01 mole de méthyl - 3 {[(méthoxy - 2 phényl) - 4 pipérazinyl - 1] - 3 méthyl - 2 propen - 1 yl} - 6 benzoxazolinone dans l'éthanol absolu. Ajouter 0,5 g de nickel de Raney et agiter sous atmosphère d'hydrogène, à température et pression ordinaires. Après absorption de la quantité théorique d'hydrogène, filtrer le milieu réactionnel, évaporer à sec le filtrat au bain-marie sous vide et recristalliser le résidu dans le cyclohexane.

Rendement : 78 %

Point de fusion : 105 °C

Caractéristiques spectrales :

Infra-rouge : 1760 $cm^{-1}$ : ν CO (O - CO - N)

Résonance magnétique nucléaire (solvant DMSO) :

δ = 0,88 ppm, doublet, 3H, CH - $CH_3$, J = 5,6 Hz

δ = 3,37 ppm, singulet, 3H, N - $CH_3$

δ = 3,84 ppm, singulet, 3H, O - $CH_3$

**EXEMPLE 24 : METHYL - 3 {[(METHOXY - 2 PHENYL) - 4 PIPERAZINYL -1] - 3 METHYL - 2 PROPYL} - 6 BENZOXAZOLINONE**

**STADE A :** Méthyl - 3 (bromo - 3 méthyl - 2 propionyl) - 6 benzoxazolinone

Dans une fiole de 250 $cm^3$ dissoudre par agitation 0,05 mole de méthyl - 3 (méthylène - 2 propionyl) - 6 benzoxazolinone obtenue dans l'exemple 15, stade A dans 150 $cm^3$ d'acétone. Faire barboter dans la solution un courant d'acide bromhydrique, maintenir l'agitation une heure. Filtrer, évaporer le filtrat au bain-marie sous vide et recristalliser le résidu dans l'éthanol absolu.

Rendement : 85 %

Point de fusion : 127 °C

**STADE B :** Méthyl - 3 (bromo - 3 méthyl - 2 propyl) - 6 benzoxazolinone

Dans une fiole de 150 $cm^3$ dissoudre 0,03 mole de méthyl - 3(bromo -3 méthyl - 2 propionyl) - 6 benzoxazolinone dans 0,3 mole d'acide trifluoroacétique. Ajouter goutte à goutte et sous refroidissement 0,066 mole de triéthylsilane. Poursuivre l'agitation pendant 24 heures à température ambiante. Verser le mélange réactionnel dans 5 volumes d'eau glacée. Essorer le précipité obtenu, laver à l'eau jusqu'à neutralité des eaux de lavage, sécher et recristalliser dans le cyclohexane.

Rendement : 67 %

Point de fusion : 70 °C

**STADE C :** Méthyl - 3 {[(méthoxy - 2 phényl) - 4 pipérazinyl - 1] -3 méthyl - 2 propyl} - 6 benzoxazolinone

Dans une fiole rodée de 250 $cm^3$ munie d'un réfrigérant à reflux dissoudre 0,02 mole de méthyl - 3 (bromo - 3 méthyl - 2 propyl) - 6 benzoxazolinone obtenue au stade B dans le dioxanne anhydre. Ajouter sous agitation magnétique 0,02 mole de (méthoxy - 2 phényl) - 1 pipérazine et 0,02 mole de triéthylamine en solution dans le dioxanne. Porter à reflux 48 heures. Essorer à chaud le précipité formé, évaporer le filtrat au bain-marie sous vide. Reprendre le résidu par 500 $cm^3$ d'eau, alcaliniser. Extraire au chloroforme, sécher les phases organiques sur chlorure de calcium. Filtrer, évaporer, le solvant au bain-marie sous vide. Recristalliser le résidu dans cyclohexane. Les caractéristiques physico-chimiques de ce produit sont en tous points identiques à celles du produit obtenu dans l'exemple 23.

**EXEMPLE 25 : METHYL - 3 {[( METHOXY - 2 PHENYL) - 4 PIPERAZINYL -1] - 3 PROPYL} - 6 BENZOXAZOLINONE**

**STADE A :** (Bromo - 3 propionyl) - 6 méthyl - 3 benzoxazolinone

Dans une fiole rodée contenant 37,4 g (0,28 mole) de chlorure d'aluminium anhydre, introduire, goutte à goutte et sous agitation 6,02 ml (0,078 mole) de diméthyl formamide.

Munir la fiole d'un réfrigérant à reflux et porter dans un bain d'huile à une température voisine de 40 - 45 ° C. Introduire 0,04 mole de méthyl - 3 benzoxazolinone et 0,044 mole de chlorure d'acide bromo - 3 propionique. Chauffer à une température voisine de 75 ° C pendant 2H30.

Après refroidissement, verser le mélange réactionnel dans 300 g de glace, acidifier par l'acide chlorhydrique concentré et agiter pendant 1H30.

Essorer le précipité obtenu, laver à l'eau et sécher. Recristalliser dans le dioxanne.

Rendement : 85 %
Point de fusion : 184 ° C (décomposition)
Caractéristiques spectrales :
Infra-rouge :
1765 $cm^{-1}$ : ν CO (O - CO - N)
1660 $cm^{-1}$ : ν CO (cétonique)
Résonance magnétique nucléaire (solvant DMSO) :
δ = 3,44 ppm, singulet, 3H, N - $CH_3$

**STADE B :** (Bromo - 3 propyl) - 6 méthyl - 3 benzoxazolinone

Dans une fiole rodée, dissoudre 0,02 mole de (bromo - 3 propionyl) -6 méthyl - 3 benzoxazolinone obtenue au stade précédent, dans 0,2 mole d'acide trifluoroacétique. Ajouter goutte à goutte et en refroidissant 0,044 mole de triéthylsilane. Adapter une garde à chlorure de calcium et poursuivre l'agitation pendant 72 heures. Verser alors le milieu réactionnel dans l'eau glacée, essorer le précipité obtenu, sécher et recristalliser dans l'hexane.

Rendement : 85 %
Point de fusion : 83-84 ° C
Caractéristiques spectrales :
Infra-rouge : 1780 $cm^{-1}$ : ν CO (O - CO - N)
Résonance magnétique nucléaire (solvant DMSO) :
δ = 2,14 ppm, multiplet, 2H, - $CH_2$ - $CH_2$ Br
δ = 2,68 ppm, triplet, 2H, - $CH_2$ - $(CH_2)_2$Br

**STADE C :** Methyl - 3 {[(méthoxy - 2 phényl) - 4 pipérazinyl - 1] -3 propyl} - 6 benzoxazolinone

Procéder comme dans l'exemple 24, stade C en remplaçant la méthyl - 3 (bromo - 3 méthyl - 2 propyl) - 6 benzoxazolinone par la méthyl - 3 (bromo - 3 propyl) - 6 benzoxazolinone obtenue au stade précédent. On obtient le produit du titre.

Solvant de recristallisation : hexane
Caractéristiques spectrales :
Infra-rouge : 1760 $cm^{-1}$ : ν CO (O - CO - N)
Résonance magnétique nucléaire (solvant DMSO) :
δ = 3,38 ppm, singulet, 3H N - $CH_3$
δ = 3,83 ppm, singulet, 3H O - $CH_3$

**EXEMPLE 26 : {[( METHOXY - 2 PHENYL) - 4 PIPERAZINYL -1] - 3 PROPYL} - 6 BENZOXAZOLINONE**

**STADE A :** (Bromo - 3 propionyl) - 6 benzoxazolinone

Procéder comme dans l'exemple 25, stade A en remplaçant la méthyl - 3 benzoxazolinone par la benzoxazolinone, on obtient le produit attendu.

Rendement : 70 %
Point de fusion : 162 ° C (décomposition)
Caractéristiques spectrales :
Infra-rouge :

3340, 3100 $cm^{-1}$ : ν NH
1755 $cm^{-1}$ : ν CO (O - CO - N)
1655 $cm^{-1}$ : ν CO (cétone)
Résonance magnétique nucléaire (solvant DMSO) :
δ = 3,41 à 4,00 ppm, multiplet, 4H, $CH_2$ - $CH_2$
δ = 11,71 ppm, signal large 1H NH échangeable

**STADE B :** (Bromo - 3 propyl) 6 benzoxazolinone

Procéder comme dans l'exemple 25, stade B en remplaçant la méthyl -3 (bromo - 3 propionyl) - 6 benzoxazolinone par la (bromo - 3 propionyl) -6 benzoxazolinone obtenue au stade A.
Rendement : 80 %
Point de fusion : 131-132 ° C (décomposition)
Caractéristiques spectrales :
Infra-rouge :
3300, 3040 $cm^{-1}$ : ν NH
1775 $cm^{-1}$ : ν CO (O - CO - N)
Résonance magnétique nucléaire (solvant DMSO) :
δ = 2,12 ppm, multiplet, 2H, $CH_2$ - $CH_2$ - $CH_2$ Br
δ = 2,66 ppm, triplet, 2H, $CH_2$ - $CH_2$ - $CH_2$ Br
δ = 3,46 ppm, multiplet, 2H, $CH_2$ - $CH_2$ - $CH_2$ Br
δ = 9,40 ppm, signal large, 1H, échangeable, NH

**STADE C :** {[(Méthoxy - 2 phényl) - 4 pipérazinyl - 1] - 3 propyl} -6 benzoxazolinone

En procédant comme dans l'exemple 25, stade C et en remplaçant la méthyl - 3 (bromo - 3 propyl) - 6 benzoxazolinone par la (bromo - 3 propyl) - 6 benzoxazolinone, on obtient le produit attendu.
Rendement : 70 %
Caractéristiques spectrales :
Infra-rouge :
3200, 2400 $cm^{-1}$ : ν NH et ν CH
1765 $cm^{-1}$ : ν CO (O - CO - N)
Résonance magnétique nucléaire (solvant DMSO) :
δ = 3,80 ppm, singulet, O $CH_3$

**EXEMPLES 27 ET 28 :**

En remplaçant dans les exemples 25 et 26 la (méthoxy - 2 phényl) - 1 pipérazine par la (fluoro - 4 phényl) - 1 pipérazine, on obtient la :
**METHYL - 3 {[(FLUORO - 4 PHENYL) - 4 PIPERAZINYL - 1] - 3 PROPYL} -6 BENZOXAZOLINONE (EXEMPLE 27)**
**{[(FLUORO - 4 PHENYL) - 4 PIPERAZINYL - 1] - 3 PROPYL} - 6 BENZOXAZOLINONE (EXEMPLE 28)**

**EXEMPLES 29 ET 30 :**

En remplaçant dans les exemples 25 et 26 la (méthoxy - 2 phényl) - 1 pipérazine par la (trifluorométhyl - 3 phényl) - 1 pipérazine, on obtient la :
**METHYL - 3 {[(TRIFLUOROMETHYL - 3 PHENYL) - 4 PIPERAZINYL -1] - 3 PROPYL} - 6 BENZOXAZOLINONE (EXEMPLE 29)**
**{[(TRIFLUOROMETHYL - 3 PHENYL) - 4 PIPERAZINYL - 1] - 3 PROPYL} - 6 BENZOXAZOLINONE (EXEMPLE 30).**

**EXEMPLE 31 : METHYL - 3 {[(PYRIDYL - 2) - 4 PIPERAZINYL - 1] - 3 OXO - 1 PROPYL} - 6**

**BENZOXAZOLINONE**

En procédant comme dans l'exemple 1, mais en remplaçant la (trifluorométhyl - 3 phényl) - 1 pipérazine par la (pyridyl - 2) - 1 pipérazine, on obtient le produit du titre.

**EXEMPLE 32 : METHYL - 3 [(BENZYL - 4 PIPERAZINYL - 1) - 3 OXO - 1 PROPYL] - 6 BENZOXAZOLINONE**

En procédant comme dans l'exemple 1, mais en remplaçant la (trifluorométhyl - 3 phényl) - 1 pipérazine par la benzyl - 1 pipérazine, on obtient le produit du titre.

**EXEMPLE 33 : METHYL - 3 {[( METHYL - 6 PYRIDYL - 2) - 4 PIPERAZINYL - 1] - 3 OXO - 1 PROPYL} - 6 BENZOXAZOLINONE**

En procédant comme dans l'exemple 1, mais en remplaçant la (trifluorométhyl - 3 phényl) - 1 pipérazine par la (méthyl - 6 pyrid - 2 yl) - 1 pipérazine, on obtient le produit du titre.

**EXEMPLE 34 : METHYL - 3 {[(PYRIMIDINYL - 2) - 4 PIPERAZINYL -1] -3 HYDROXY - 1 PROPYL} - 6 BENZOXAZOLINONE**

En procédant comme dans l'exemple 5, mais en remplaçant la méthyl -3 {[(trifluorométhyl - 3 phényl) - 4 pipérazinyl - 1] - 3 oxo - 1 propyl} - 6 benzoxazolinone par la méthyl - 3 {[(pyrimidinyl - 2) - 4 pipérazinyl - 1] - 3 oxo - 1 propyl} - 6 benzoxazolinone obtenue dans l'exemple 4, on obtient le produit du titre.

**EXEMPLE 35 : METHYL - 3 [(PYRIMIDINYL - 2) - 4 PIPERAZINYL - 1] -3 PROPEN - 1 YL} - 6 BENZOXAZOLINONE**

En procédant comme dans l'exemple 8, mais en remplaçant la méthyl -3 {[(trifluorométhyl - 3 phényl) - 4 - pipérazinyl - 1] - 3 hydroxy - 1 propyl} - 6 benzoxazolinone par la méthyl - 3 {[(pyrimidinyl - 2) - 4 pipérazinyl - 1] - 3 hydroxy - 1 propyl} - 6 benzoxazolinone obtenue dans l'exemple 34, on obtient le produit du titre.

**EXEMPLE 36 : METHYL - 3 {[(METHYL - 6 PYRIDYL - 2) - 4 PIPERAZINYL - 1] - 3 HYDROXY - 1 PROPYL} - 6 BENZOXAZOLINONE**

En procédant comme dans l'exemple 5, mais en remplaçant la méthyl -3 {[(trifluorométhyl - 3 phényl) - 4 pipérazinyl - 1] - 3 oxo - 1 propyl} - 6 benzoxazolinone par la méthyl - 3 {[(méthyl - 6 pyridyl - 2) - 4 pipérazinyl - 1] - 3 oxo - 1 propyl} - 6 benzoxazolinone obtenue dans l'exemple 33, on obtient le produit du titre.

**EXEMPLE 37 : METHYL - 3 {[(METHYL - 6 PYRIDYL - 2) - 4 PIPERAZINYL - 3 PROPEN - 1 - YL] - 6 BENZOXAZOLINONE**

En procédant comme dans l'exemple 8, mais en remplaçant la méthyl -3 {[(trifluorométhyl - 3 phényl) - 4 pipérazinyl - 1] - 3 hydroxy - 1 propyl} - 6 benzoxazolinone par la méthyl - 3 {[(méthyl - 6 pyridyl - 2) -

**EXEMPLE 38 : METHYL - 3 [(PHENYL - 4 PIPERAZINYL - 1) - 3 OXO - 1 PROPYL] - 6 BENZOXAZOLINONE**

En procédant comme dans l'examplе 1, mais en remplaçant la (trifluorométhyl - 3 phényl) - 1 pipérazine par la phényl - 1 pipérazine, on obtient le produit du titre.

**EXEMPLE 39 : METHYL 3 [(PHENYL - 4 PIPERAZINYL - 1] - 3 HYDROXY - 1 PROPYL] - 6 BENZOXAZOLINONE**

En procédant comme dans l'example 5 mais en remplaçant la méthyl - 3 {[(trifluorométhyl - 3 phényl) - 4 pipérazinyl - 1] - 3 oxo - 1 propyl} -6 benzoxazolinone par la méthyl - 3 [(phényl - 4 pipérazinyl - 1) - 3 oxo - 1 propyl] - 6 benzoxazolinone obtenue dans l'exemple 38, on obtient le produit du titre.

**EXEMPLE 40 : METHYL - 3[(PHENYL - 4 PIPERAZINYL - 1) - 3 PROPYL] -6 BENZOXAZOLINONE**

En procédant comme dans l'example 25 et en remplaçant la (méthoxy -2 phényl) - 1 pipérazine par la phényl - 1 pipérazine, on obtient le produit du titre.

**EXEMPLE 41 : [(PHENYL - 4 PIPERAZINYL - 1) - 3 PROPYL] - 6 BENZOXAZOLINONE**

En procédant comme dans l'example 26 et en remplaçant la méthoxy -2 phényl) - 1 pipérazine par la phényl - 1 pipérazine, on obtient le produit du titre.

**EXEMPLE 42 : METHYL - 3 {[(PYRIDYL - 2) - 4 PIPERAZINYL - 1] - 3 HYDROXY} - 1 PROPYL - 6 BENZOXAZOLINONE**

En procédant comme dans l'example 5, mais en remplaçant la (méthyl -3 {[(trifluorométhyl - 3 phényl) - 4 pipérazinyl - 1] - 3 oxo - 1 propyl} - 6 benzoxazolinone par la méthyl - 3 {[(pyridyl - 2) - 4 pipérazinyl - 1] - 3 oxo - 1 propyl} - 6 benzoxazolinone obtenue dans l'exemple 31, on obtient le produit du titre.

**EXEMPLE 43 : METHYL - 3 {[(PYRIDYL - 2) - 4 PIPERAZINYL - 1] - 3 PROPYL} - 6 BENZOXAZOLINONE**

En procédant comme dans l'exemple 25 et en remplaçant la (méthoxy -2 phényl) - 1 pipérazine par la (pyridyl - 2) - 1 pipérazine, on obtient le produit du titre.

**EXEMPLE 44 : {[(PYRIDYL - 2) - 4 PIPERAZINYL - 1] -3 PROPYL} - 6 BENZOXAZOLINONE**

En procédant comme dans l'example 26 et en remplaçant la (méthoxy -2 phényl) - 1 pipérazine par la (pyridyl - 2) - 1 pipérazine, on obtient le produit du titre.

## ETUDE PHARMACOLOGIQUE DES DERIVES DE L'INVENTION

**EXEMPLE 45 : ETUDE DE LA TOXICITE AIGUE**

La toxicité aiguë a été appréciée après administration orale à des lots de 8 souris (26±2 grammes) d'une dose de 1000 $mg.kg^{-1}$. Les animaux ont été observés à intervalles réguliers au cours de la première journée et quotidiennement pendant les 2 semaines suivant le traitement.

Il apparaît que la toxicité des dérivés de l'invention est très variable selon leur structure. Ceux pour lesquels X et Y représentent simultanément un atome d'oxygène ont une toxicité très faible (0% de décès). Pour les autres dérivés, le pourcentage de décès obtenu est très variable selon la nature de X, Y et Ar.

**EXEMPLE 46 : ETUDE DE L'ACTIVITE ANALGESIQUE**

L'activité sur la douleur a été recherchée chez la souris (23-25 g) selon un protocole dérivé de la

technique décrite par SIEGMUND (SIEGMUND E.A., R.A. CADMUS & GOLU, J. Pharm. Exp. Ther. 119, 1874, 1957). Les souris, réparties par randomisation en lots de 12 animaux, ont reçu le traitement par voir orale (excipient pour les témoins) 1 heure avant l'injection intra-péritonéale d'une solution hydroalcoolique de phényl-p-benzoquinone (Sigma) à 0,02 %. Les étirements sont dénombrés entre la 5ème et 10ème minute après l'injection.

Le pourcentage d'activité obtenu a été évalué pour chaque dose (% de diminution du nombre d'étirements chez les traités par rapport aux témoins). Une $ED_{50}$, dose entraînant une activité de 50 %, a été déterminée pour chaque produit.

Il est apparu que certains composés de l'invention possèdent une activité analgésique très intéressante. Ainsi, l'$ED_{50}$ du composé de l'exemple 3 est voisine de 2 $mg.kg^{-1}$; l'$ED_{50}$ de l'exemple 2 est voisine de 5 $mg.kg^{-1}$.

A titre de comparaison l'administration d'une dose de 100 $mg.kg^{-1}$ des dérivés du brevet Fr 73.23280 provoquait un pourcentage d'analgésie -dans un test comparable - de l'ordre de 25 à 60 % et le composé du brevet Fr 80.20861 dont l'activité analgésique est la plus intéressante avait dans ce même test de Siegmund une $ED_{50}$ de 9 $mg.kg^{-1}$ soit 4,5 fois supérieure à celle du produit le plus intéressant de la présente invention.

**EXEMPLE 47: ETUDE DE L'ACTIVITE ANTI-INFLAMMATOIRE**

Le potentiel anti-inflammatoire des composés a été recherché sur un modèle d'inflammation aiguë provoquée par injection sous-cutanée d'une solution de carragénine au niveau de la patte postérieure de rat, selon une technique inspirée de la méthode de WINTERCH ; E.A. RISLEY, G.N. NUSS - (Proc. Soc. Exp. Med. 111, 554, 1962). Les rats (100-120 g), randomisés en lots de 8, ont été traités (y compris les témoins qui reçoivent l'excipient) 1 heure avant l'injection locale d'une suspension à 0,5 % de carragénine (type IV, Sigma ; 0,1 ml par rat). L'oedème est déterminé 3 heures après l'injection, par mesure pléthysmométrique (pléthysmomètre à eau UGO BASILE) du volume de chacune des pattes postérieures (oedème = volume patte enflammée - volume patte non enflammée).

Il apparaît que les produits de l'invention n'ont aucune activité sur ce test ou une activité très faible. En comparaison, les produits du brevet Fr 73.23280 possèdent une activité anti-inflammatoire.

| **EXEMPLE 48 : COMPOSITION PHARMACEUTIQUE : COMPRIME** | |
|---|---|
| Comprimés dosés à 20 mg de méthyl - 3 {[(fluoro - 4 phényl) - 4 pipérazinyl - 1] - 3 oxo - 1 propyl} - 6 benzoxazolinone. | |
| Formule de préparation pour 1000 comprimés. | |
| Méthyl - 3 {[(fluoro - 4 phényl) - 4 pipérazinyl - 1] - 3 oxo - 1 propyl} - 6 benzoxazolinone | 20 g |
| Amidon de blé | 15 g |
| Amidon de maïs | 15 g |
| Lactose | 65 g |
| Stéarate de magnésium | 2 g |
| Silice | 1 g |
| Hydroxypropylcellulose | 2 g |

## Revendications

1/ Composés de formule générale (I) :

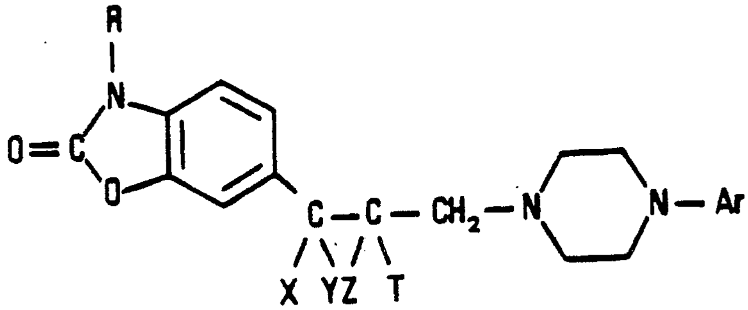

(I)

dans laquelle :

X représente un atome d'hydrogène,

Y représente un atome d'hydrogène ou un groupement hydroxyle ou bien X et Y représentent ensemble un atome d'oxygène,

T représente un atome d'hydrogène ou un groupement alcoyle inférieur,

Z représente un atome d'hydrogène ou bien Z forme avec Y une liaison π , et dans ce cas X représente un atome d'hydrogène,

R représente un atome d'hydrogène ou un groupement alcoyle inférieur,

Ar représente un groupement aryle, ou hétéroaryle, ou alkyl inférieur aryle, éventuellement substitué par un atome d'halogène, un groupement alcoyle ou alcoyloxy inférieur, eux-mêmes éventuellement substitués par un ou plusieurs atomes d'halogène,

leurs énantiomères, épimères, diastéréoisomères,

ainsi que leurs sels d'addition à un acide pharmaceutiquement acceptable.

2/ Composés selon la revendication 1, dans lesquels X et Y simultanément représentent ensemble un atome d'oxygène ainsi que leurs sels d'addition à un acide pharmaceutiquement acceptable.

3/ Composés selon la revendication 1, dans lesquels X représente un atome d'hydrogène et Y un groupement hydroxyle, leurs énantiomères, épimères, diastéréoisomères ainsi que leurs sels d'addition à un acide pharmaceutiquement acceptable.

4/ Composé selon la revendication 1, dans lesquels X et Y représentent simultanément chacun un atome d'hydrogène ainsi que leurs sels d'addition à un acide pharmaceutiquement acceptable.

5/ Composé selon la revendication 1, dans lesquels Y forme avec Z une liaison π , leurs isomères ainsi que leurs sels d'addition à un acide pharmaceutiquement acceptable.

6/ Composés selon la revendication 1, dans lesquels Ar représente le groupement phényle, leurs énantiomères, épimères, diastéréoisomères ainsi que leurs sels d'addition à un acide pharmaceutiquement acceptable.

7/ Composés selon la revendication 1, dans lesquels Ar représente un groupement phényle substitué par un atome d'halogène, un groupement alkoxy inférieur ou un groupement trifluorométhyle, leurs énantiomères, épimères, diastéréoisomères ainsi que leurs sels d'addition à un acide pharmaceutiquement acceptable.

8/ Composés selon la revendication 1, dans lesquels Ar représente un radical pyridyle - 2 ou pyrimidinyle - 2, leurs énantiomères, épimères, diastéréoisomères ainsi que leurs sels d'addition à un acide pharmaceutiquement acceptable.

9/ Composé selon l'une des revendications 1 et 2 qui est la méthyl - 3 {[(fluoro - 4 phényl) - 4 pipérazinyl - 1] - 3 oxo - 1 propyl} - 6 benzoxazolinone, ainsi que ses sels d'addition à un acide pharmaceutiquement acceptable.

10/ Composé selon l'une des revendications 1 et 2 qui est la méthyl - 3 {[(méthoxy - 2 phényl) - 4 pipérazinyl - 1] - 3 oxo - 1 propyl} - 6 benzoxazolinone, ainsi que ses sels d'addition à un acide pharmaceutiquement acceptable.

11/ Procédé de préparation des composés de formule (I) caractérisé en ce que l'on utilise comme matière première un dérivé de formule (II) :

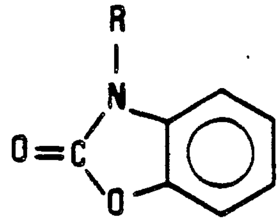

(II)

dans laquelle, R a la même signification que dans la formule (I), que l'on soumet à l'action d'un chlorure d'acide de formule (III) :

$Cl\text{-}CO\text{-}\underset{T}{\underset{|}{HC}}\text{-}CH_2\text{-}A$ (III)

dans laquelle, T a la même signification que dans la formule (I), A représentant un atome d'halogène, ou bien de l'anhydride d'acide correspondant, en présence de chlorure d'aluminium en milieu de diméthyl formamide, pour obtenir un dérivé de formule (IV) :

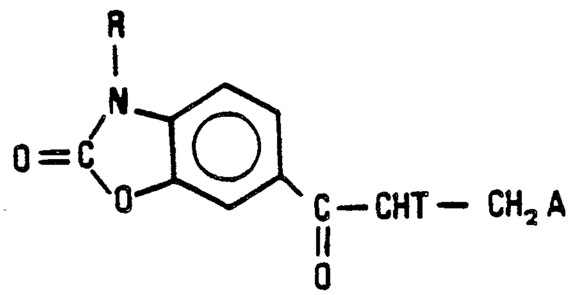

(IV)

dans laquelle R et T ont la même signification que dans la formule (I) et A la même signification que dans la formule (III), qui, si on le désire, est soumis à réduction par un trialkylsilane en milieu acide, pour conduire à un dérivé de formule (V) :

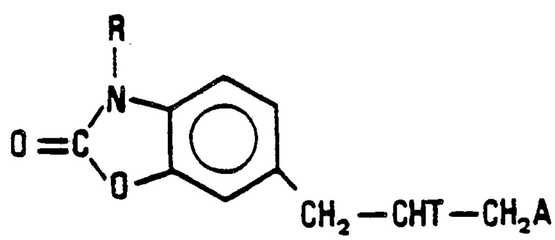

(V)

dans laquelle R et T ont la même signification que dans la formule (I), et A la même signification que dans la formule (III), le dérivé de formule (IV), ou le dérivé de formule (V), selon la formule du dérivé de formule (I) que l'on souhaite obtenir, étant alors soumis à l'action d'une aryl - 1 pipérazine de formule (VI) :

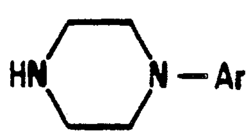

(VI)

dans laquelle Ar a la même signification que dans la formule (I), dans un solvant préférentiellement choisi parmi acétone, acétonitrile, acétate d'éthyle, alcool aliphatique inférieur, dioxanne, benzène, toluène, à une température comprise entre la température ambiante et la température d'ébullition du solvant choisi, en présence d'un excès de l'amine choisie ou d'un capteur de l'hydracide formé tel que la triéthylamine,

pour conduire à un dérivé de formule (I/A) :

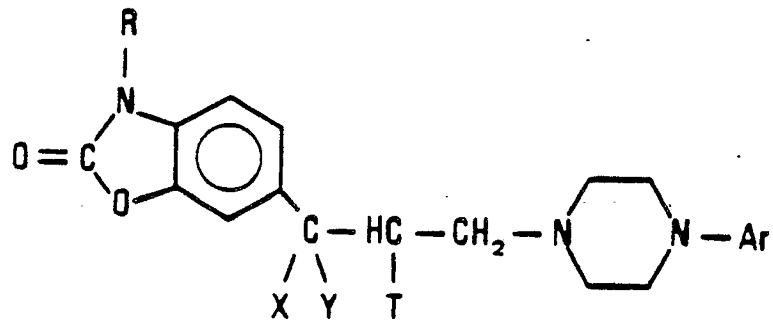

(I/A)

dans laquelle, selon que la matière première utilisée est un dérivé de formule (IV) ou (V),

X et Y représentent ensemble un atome d'oxygène, ou bien X et Y représentent tous deux simultanément un atome d'hydrogène,

R, Ar et T ayant la même signification que dans la formule (I),

qui, si on le désire, est salifié par un acide pharmaceutiquement acceptable ou qui peut, lorsque X et Y représentent ensemble un atome d'oxygène, si on le désire, être soumis,

* ou bien, à un agent d'hydrogénation choisi parmi un hydrure mixte de métal alcalin, comme par exemple le borohydrure de sodium, ou un cyanohydrure mixte de métal alcalin, comme le cyano borohydrure de sodium, préférentiellement en milieu d'alcool aliphatique inférieur, pour conduire à un dérivé de formule (I/B) - majoritairement sous la configuration thréo lorsque T ne représente pas un atome d'hydrogène - :

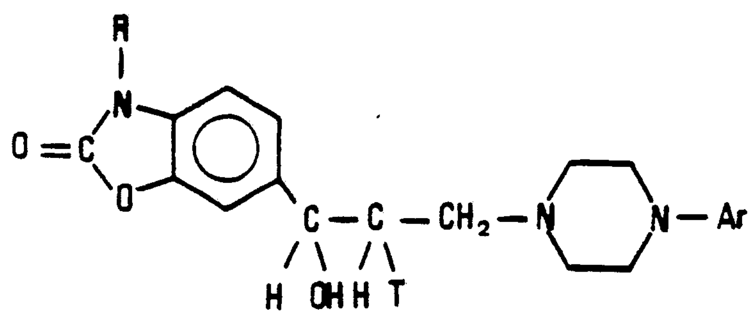

(I/B)

cas particulier des dérivés de formule (I), dans laquelle :

R, T et Ar ont la même signification que dans les dérivés de formule (I),

X représentant ici un atome d'hydrogène,

Y un groupement hydroxyle et Z un atome d'hydrogène,

dont on sépare, si on le désire, les isomères et/ou que l'on salifie par un acide pharmaceutiquement acceptable,

* ou bien à hydrogénation catalytique, sous chauffage et pression dans un solvant choisi parmi alcool aliphatique inférieur ou dioxanne, pour conduire à un dérivé de formule (I/B) - essentiellement sous la configuration érythro lorsque T ne représente pas un atome d'hydrogène - dont on sépare, si on le désire, les isomères et que l'on salifie le cas échéant par un acide pharmaceutiquement acceptable,

dérivé de formule (I/B) qui, quelque soit le procédé selon lequel il a été obtenu peut être, si on le désire, traité par un agent déshydratant,

préférentiellement choisi parmi les hydracides, pour conduire à un dérivé de formule (I/C) :

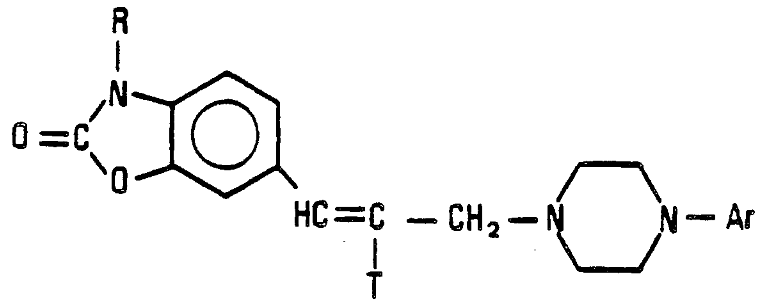

(I/C)

cas particulier de dérivés de formule (I), dans laquelle :

R, T et Ar ont la même signification que dans les dérivés de formule (I),

X représentant ici un atome d'hydrogène,

Z formant avec Y une liaison π,

dont, on sépare, si on le désire, les isomères cis-trans par une technique choisie parmi cristallisation ou chromatographie,

et qui, si on le souhaite, peut être salifié par un acide pharmaceutiquement acceptable.

12/ Procédé de préparation des dérivés de formule (I) pour lesquels R représente un groupement alcoyle inférieur caractérisé én ce que l'on acyle un dérivé de formule (II) :

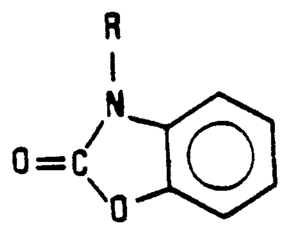

(II)

dans laquelle R représente un groupement alcoyle inférieur par un acide de formule (VII) :

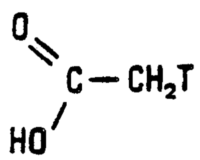

(VII)

dans laquelle T a la même signification que dans la formule (I) ou le chlorure ou l'anhydride de l'acide correspondant en présence d'acide polyphosphonique,

pour obtenir un dérivé de formule (VIII) :

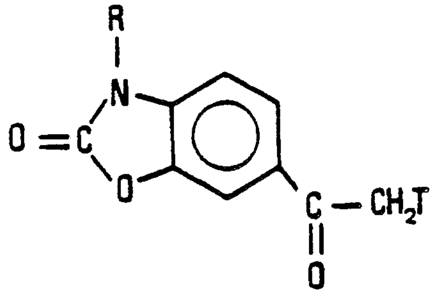

(VIII)

dans laquelle :

R représente un groupement alcoyle inférieur, et T a la même signification que dans la formule (I),

que l'on traite ensuite,

* ou bien, selon les conditions de la réaction de Mannich, bien connues de l'homme de l'Art, en présence

de trioxyméthylène et de l'aryl pipérazine choisie de formule (VI) :

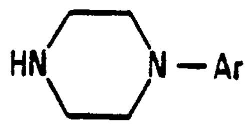

(VI)

dans laquelle, Ar a la même signification que dans la formule (I), pour obtenir un dérivé de formule (I/A1) :

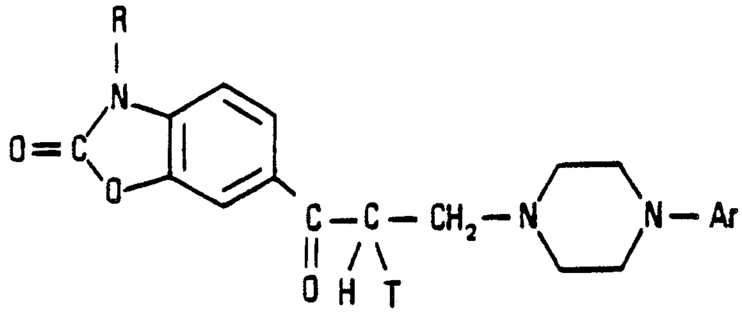

(I/A1)

cas particulier des dérivés de formules (I/A) et (I), dans laquelle :

R représente un groupement alcoyle inférieur, et T et Ar ont la même signification que dans la formule (I),

X et Y représentant ici simultanément un atome d'oxygène et Z un atome d'hydrogène,

* ou bien par le bisdiméthylamino méthane en milieu d'anhydride acétique pour obtenir un produit de formule générale (IX) :

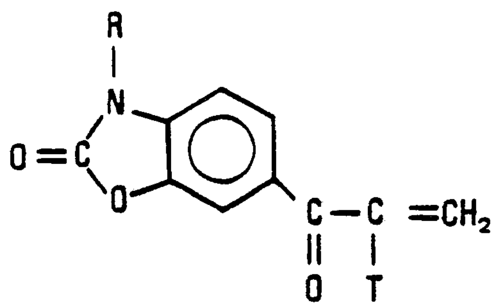

(IX)

dans laquelle :

T a la même signification que dans la formule (I) et R représente un groupement alcoyle inférieur,

que l'on traite par une amine de formule (VI), dans un solvant polaire à une température comprise entre la température ambiante et la température d'ébullition du milieu réactionnel,

pour conduire à un dérivé de formule (I/A1) précédemment défini,

qui, lorsque T ne représente pas un atome d'hydrogène peut être, si on le désire, séparé en ses énantiomères, que l'on salifie, si on le désire par un acide pharmaceutiquement acceptable et qui,

si on le souhaite, peut être soumis,

* ou bien préférentiellement en milieu d'alcool aliphatique inférieur à un agent d'hydrogénation préférentiellement un hydrure mixte de métal alcalin ou un cyanohydrure mixte de métal alcalin comme par exemple le borohydrure de sodium, ou le cyanoborohydrure de sodium,

pour conduire à un dérivé de formule (I/B) majoritairement sous la configuration thréo (lorsque T ne représente pas un atome d'hydrogène) :

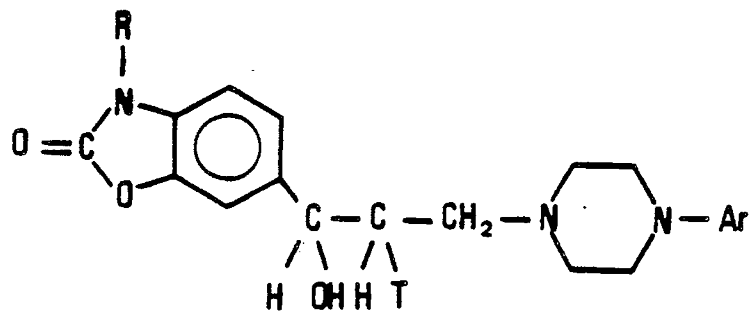

(I/B)

cas particulier des dérivés de formule (I), dans laquelle :
R, T et Ar ont la même signification que dans les dérivés de formule (I),
X représentant ici un atome d'hydrogène,
Y un groupement hydroxyle et Z un atome d'hydrogène,
dont on sépare, si on le désire, les isomères et que l'on peut salifier par un acide pharmaceutiquement acceptable,
* ou bien à hydrogénation catalytique dans un solvant choisi parmi alcool, aliphatique inférieur ou dioxanne pour conduire à un dérivé de formule (I/B) essentiellement sous la configuration érythro -lorsque T ne représente pas un atome d'hydrogène - dont on sépare, si on le désire, les énantiomères et que l'on salifie le cas échéant par un acide pharmaceutiquement acceptable,
dérivé de formule (I/B) que, le cas échéant on soumet à un agent déshydratant choisi parmi les hydracides pour conduire à un dérivé de formule (I/C) :

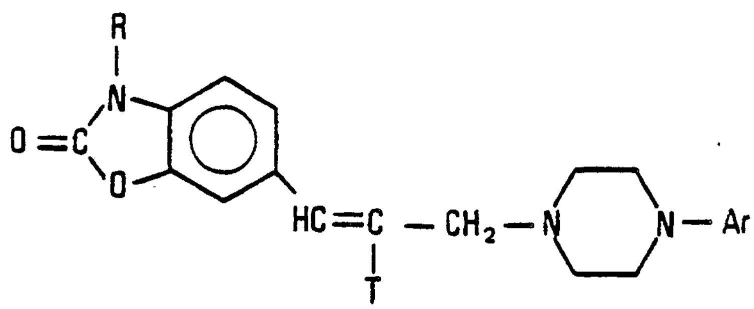

(I/C)

cas particulier de dérivés de formule (I), dans laquelle :
R, T et Ar ont la même signification que dans les dérivés de formule (I),
X représentant ici un atome d'hydrogène,
Z formant avec Y une liaison $\pi$,
dont, si on le désire, on sépare les isomères cis-trans par une technique choisie parmi cristallisation ou chromatographie,
et que l'on salifie, si on le désire, par un acide pharmaceutiquement acceptable,
qui, si on le désire, est soumis à une réaction d'hydrogénation catalytique préférentiellement à température et pression ambiantes, et en présence de Nickel de Raney en milieu d'alcool aliphatique inférieur ou de dioxanne pour obtenir un dérivé de formule (I/D) :

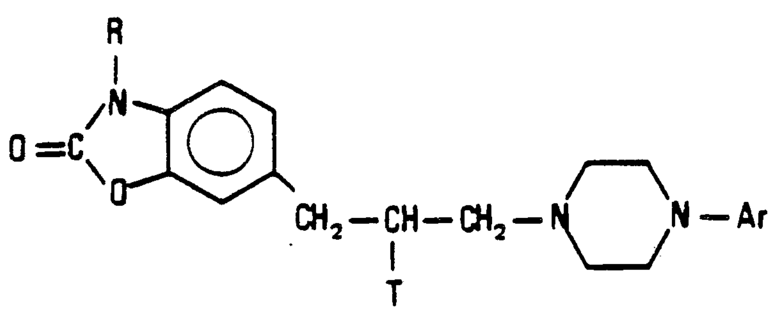

(I/D)

dans laquelle :
R, T et Ar ont la même signification que dans la formule (I),
X, Y et Z représentant chacun simultanément un atome d'hydrogène.
dont on sépare le cas échéant les isomères lorsque T ne représente pas un atome d'hydrogène,
et que l'on salifie éventuellement par un acide pharmaceutiquement acceptable.

13/ Procédé de préparation des dérivés de formule (I/D), selon la revendication 12 à partir des dérivés de formule (IX) :

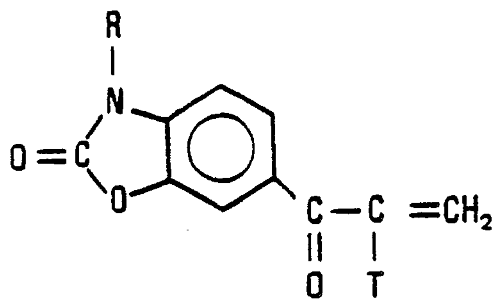

(IX)

dans laquelle :
R et T ont la même signification que dans la formule (I),
que l'on traite par un agent d'halogénation pour obtenir un dérivé de formule (IV) :

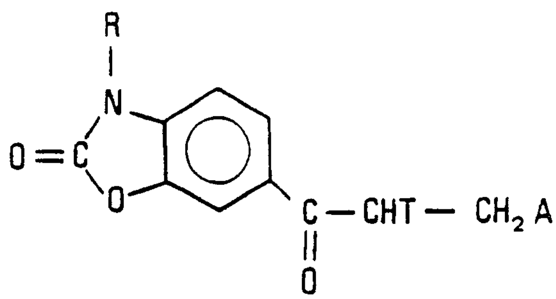

(IV)

dans laquelle R et T ont la même signification que dans la formule (I) et A la même signification que dans la formule (III),
dont on sépare si on le désire, les isomères, lorsque H ne représente pas un atome d'hydrogène,
que l'on soumet à réduction par un trialkylsilane en milieu pour conduire à un dérivé de formule (V) :

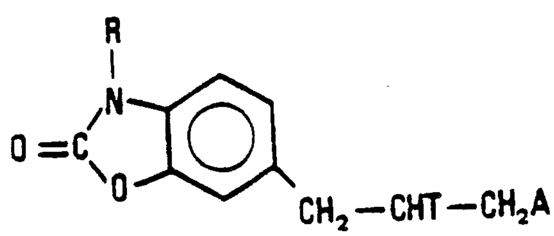

(V)

dans laquelle R et T ont la même signification que dans la formule (I), et A la même signification que dans la formule (III),
que l'on soumet à l'action d'une aryl - 1 pipérazine de formule (VI) :

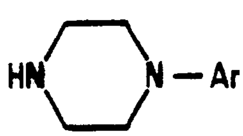

(VI)

dans laquelle Ar a la même signification que dans la formule (I), dans un solvant préférentiellement choisi parmi acétone, acétonitrile, acétate d'éthyle, alcool aliphatique inférieur, dioxanne, benzène, toluène, à une température comprise entre la température ambiante et la température d'ébullition du solvant choisi en présence d'un excès de l'amine choisie ou d'un capteur de l'hydracide formé tel que la triéthylamine, pour conduire à un dérivé de formule (I/D) selon la revendication 12,
et que l'on salifie, si on le désire par un acide pharmaceutiquement acceptable.

14/ Composition pharmaceutique contenant comme principe actif au moins un composé selon l'une des revendications 1 à 10 en combinaison avec un ou plusieurs excipients ou véhicules inertes, non-toxiques, pharmaceutiquement acceptables.

15/ Composition pharmaceutique selon la revendication 14 contenant au moins un principe actif selon l'une des revendications 1 à 10 utilisable dans le traitement d'algies.

Revendications pour l'Etat contractant suivant: GR

1/ Procédé de préparation des composés de formule générale (I) :

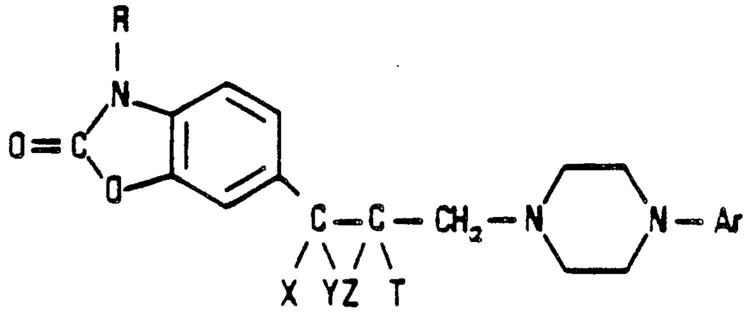

(I)

dans laquelle :
X représente un atome d'hydrogène,
Y représente un atome d'hydrogène ou un groupement hydroxyle ou bien X et Y représentent ensemble un atome d'oxygène,
T représente un atome d'hydrogène ou un groupement alcoyle inférieur,
Z représente un atome d'hydrogène ou bien Z forme avec Y une liaison $\pi$ , et dans ce cas X représente un atome d'hydrogène,
R représente un atome d'hydrogène ou un groupement alcoyle inférieur,
Ar représente un groupement aryle, ou hétéroaryle, ou alkyl inférieur aryle, éventuellement substitué par un atome d'halogène, un groupement alcoyle ou alcoyloxy inférieur, eux-mêmes éventuellement substitués par un ou plusieurs atomes d'halogène,
leurs énantiomères, épimères, diastéréoisomères,
ainsi que leurs sels d'addition à un acide pharmaceutiquement acceptable,
caractérisé en ce que l'on utilise comme matière première un dérivé de formule (II) :

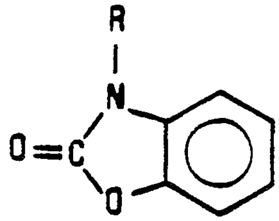

(II)

dans laquelle, R a la même signification que dans la formule (I),
que l'on soumet à l'action d'un chlorure d'acide de formule (III) :

**$Cl-CO-H\underset{T}{\underset{|}{C}}-CH_2-A$** (III)

dans laquelle, T a la même signification que dans la formule (I), A représentant un atome d'halogène, ou bien de l'anhydride d'acide correspondant,

en présence de chlorure d'aluminium en milieu de diméthyl formamide, pour obtenir un dérivé de formule (IV) :

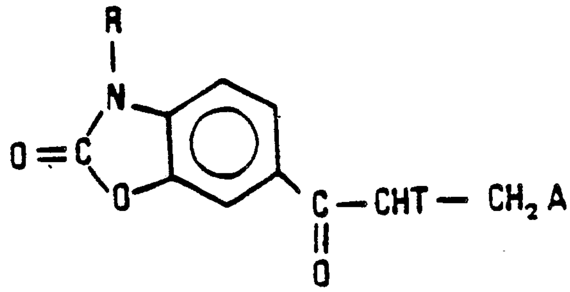

(IV)

dans laquelle R et T ont la même signification que dans la formule (I) et A la même signification que dans la formule (III), qui, si on le désire, est soumis à réduction par un trialkylsilane en milieu acide, pour conduire à un dérivé de formule (V) :

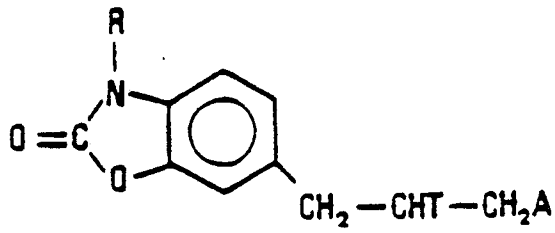

(V)

dans laquelle R et T ont la même signification que dans la formule (I), et A la même signification que dans la formule (III), le. dérivé de formule (IV), ou le dérivé de formule (V), selon la formule du dérivé de formule (I) que l'on souhaite obtenir, étant alors soumis à l'action d'une aryl - 1 pipérazine de formule (VI) :

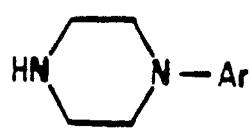

(VI)

dans laquelle Ar a la même signification que dans la formule (I), dans un solvant préférentiellement choisi parmi acétone, acétonitrile, acétate d'éthyle, alcool aliphatique inférieur, dioxanne, benzène, toluène, à une température comprise entre la température ambiante et la température d'ébullition du solvant choisi, en présence d'un excès de l'amine choisie ou d'un capteur de l'hydracide formé tel que la triéthylamine, pour conduire à un dérivé de formule (I/A) :

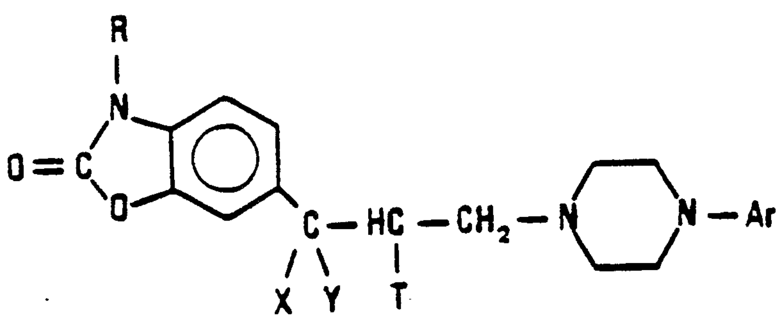

(I/A)

dans laquelle, selon que la matière première utilisée est un dérivé de formule (IV) ou (V),

X et Y représentent ensemble un atome d'oxygène, ou bien X et Y représentent tous deux simultanément un atome d'hydrogène,

R, Ar et T ayant la même signification que dans la formule (I),

qui, si on le désire, est salifié par un acide pharmaceutiquement acceptable ou qui peut, lorsque X et Y représentent ensemble un atome d'oxygène, si on le désire, être soumis,

* ou bien, à un agent d'hydrogénation choisi parmi un hydrure mixte de métal alcalin, comme par exemple le borohydrure de sodium, ou un cyanohydrure mixte de métal alcalin, comme le cyano borohydrure de sodium, préférentiellement en milieu d'alcool aliphatique inférieur, pour conduire à un dérivé de formule (I/B) - majoritairement sous la configuration thréo lorsque T ne représente pas un atome d'hydrogène - :

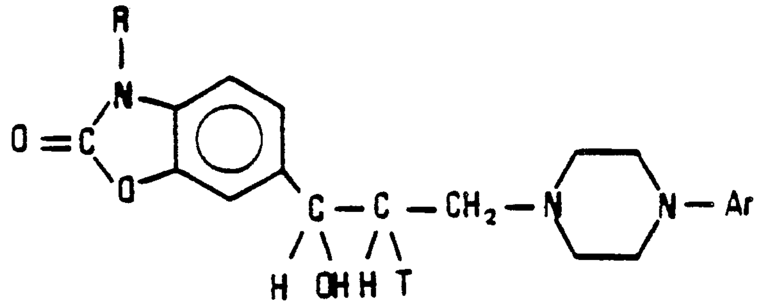

(I/B)

cas particulier des dérivés de formule (I), dans laquelle :

R, T et Ar ont la même signification que dans les dérivés de formule (I),

X représentant ici un atome d'hydrogène,

Y un groupement hydroxyle et Z un atome d'hydrogène,

dont on sépare, si on le désire, les isomères et/ou que l'on salifie par un acide pharmaceutiquement acceptable,

* ou bien à hydrogénation catalytique, sous chauffage et pression dans un solvant choisi parmi alcool aliphatique inférieur ou dioxanne, pour conduire à un dérivé de formule (I/B) - essentiellement sous la configuration érythro lorsque T ne représente pas un atome d'hydrogène - dont on sépare, si on le désire, les isomères et que l'on salifie le cas échéant par un acide pharmaceutiquement acceptable,

dérivé de formule (I/B) qui, quelque soit le procédé selon lequel il a été obtenu peut être, si on le désire, traité par un agent déshydratant,

préférentiellement choisi parmi les hydracides, pour conduire à un dérivé de formule (I/C) :

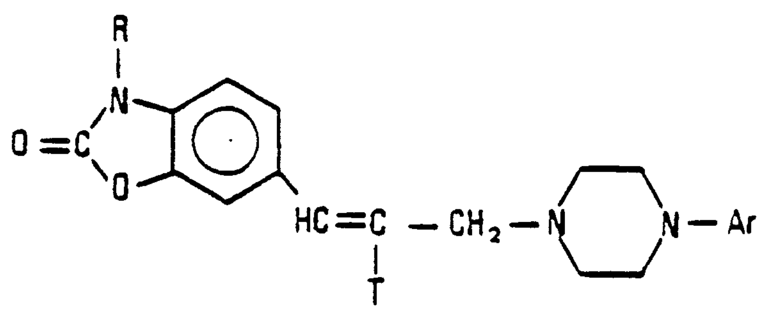

(I/C)

cas particulier de dérivés de formule (I), dans laquelle :

R, T et Ar ont la même signification que dans les dérivés de formule (I),

X représentant ici un atome d'hydrogène,

Z formant avec Y une liaison $\pi$,

dont, on sépare, si on le désire, les isomères cis-trans par une technique choisie parmi cristallisation ou chromatographie,

et que, si on le souhaite, peut être salifié par un acide pharmaceutiquement acceptable.

2/ Procédé de préparation des dérivés de formule (I) pour lesquels R représente un groupement alcoyle inférieur caractérisé en ce que l'on acyle un dérivé de formule (II) :

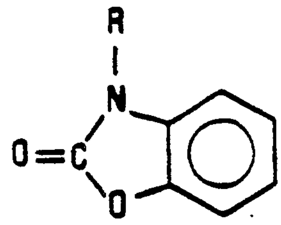

(II)

dans laquelle R représente un groupement alcoyle inférieur par un acide de formule (VII) :

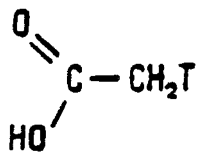

(VII)

dans laquelle T a la même signification que dans la formule (I) ou le chlorure ou l'anhydride de l'acide correspondant en présence d'acide polyphosphonique,
pour obtenir un dérivé de formule (VIII) :

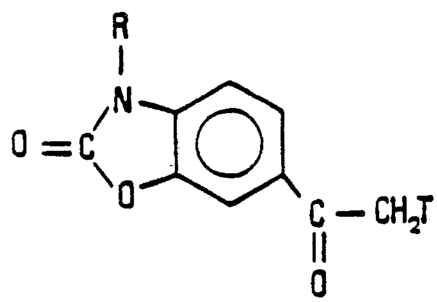

(VIII)

dans laquelle :
R représente un groupement alcoyle inférieur, et T a la même signification que dans la formule (I),
que l'on traite ensuite,
* ou bien, selon les conditions de la réaction de Mannich, bien connues de l'homme de l'Art, en présence de trioxyméthylène et de l'aryl pipérazine choisie de formule (VI) :

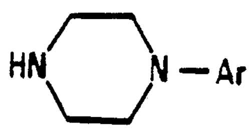

(VI)

dans laquelle, Ar a la même signification que dans la formule (I),
pour obtenir un dérivé de formule (I/A1) :

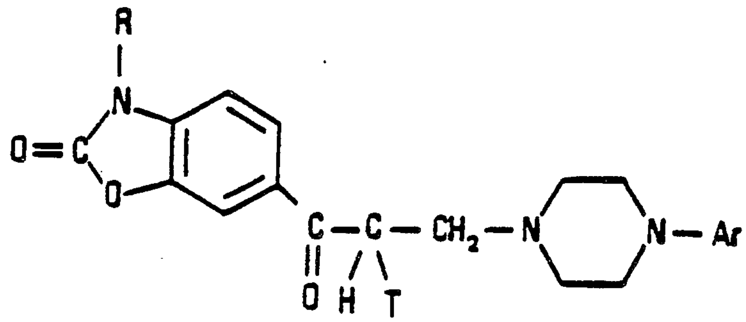

(I/A1)

cas particulier des dérivés de formules (I/A) et (I), dans laquelle :
R représente un groupement alcoyle inférieur, et T et Ar ont la même signification que dans la formule (I),
X et Y représentant ici simultanément un atome d'oxygène et Z un atome d'hydrogène,
* ou bien par le bisdiméthylamino méthane en milieu d'anhydride acétique pour obtenir un produit de formule générale (IX) :

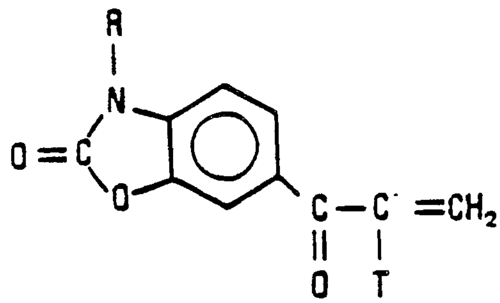

(IX)

dans laquelle :
T a la même signification que dans la formule (I) et R représente un groupement alcoyle inférieur,
que l'on traite par une amine de formule (VI), dans un solvant polaire à une température comprise entre la température ambiante et la température d'ébullition du milieu réactionnel,
pour conduire à un dérivé de formule (I/A1) précédemment défini,
qui, lorsque T ne représente pas un atome d'hydrogène peut être, si on le désire, séparé en ses énantiomères, que l'on salifie, si on le désire par un acide pharmaceutiquement acceptable et qui,
si on le souhaite, peut être soumis,
* ou bien préférentiellement en milieu d'alcool aliphatique inférieur à un agent d'hydrogénation préférentiellement un hydrure mixte de métal alcalin ou un cyanohydrure mixte de métal alcalin comme par exemple le borohydrure de sodium, ou le cyanoborohydrure de sodium,
pour conduire à un dérivé de formule (I/B) majoritairement sous la configuration thréo (lorsque T ne représente pas un atome d'hydrogène) :

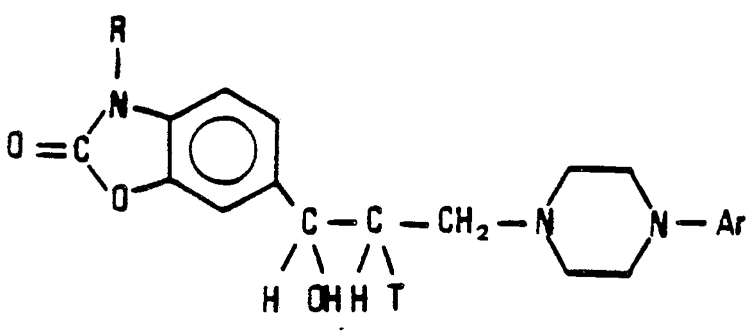

(I/B)

cas particulier des dérivés de formule (I), dans laquelle :
R, T et Ar ont la même signification que dans les dérivés de formule (I),
X représentant ici un atome d'hydrogène,

Y un groupement hydroxyle et Z un atome d'hydrogène,
dont on sépare, si on le désire, les isomères et que l'on peut salifier par un acide pharmaceutiquement acceptable,
* ou bien à hydrogénation catalytique dans un solvant choisi parmi alcool, aliphatique inférieur ou dioxanne pour conduire à un dérivé de formule (I/B) essentiellement sous la configuration érythro -lorsque T ne représente pas un atome d'hydrogène - dont on sépare, si on le désire, les énantiomères et que l'on salifie le cas échéant par un acide pharmaceutiquement acceptable,
dérivé de formule (I/B) que, le cas échéant on soumet à un agent déshydratant choisi parmi les hydracides pour conduire à un dérivé de formule (I/C) :

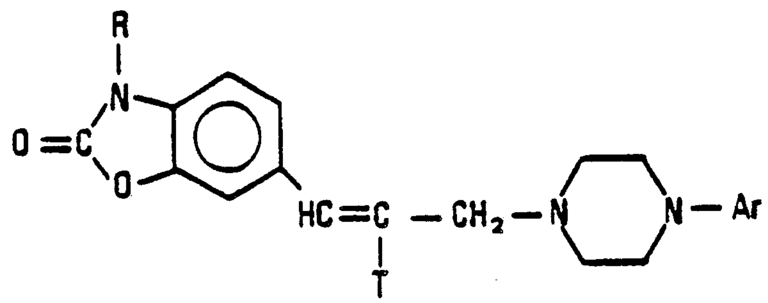

(I/C)

cas particulier de dérivés de formule (I), dans laquelle :
R, T et Ar ont la même signification que dans les dérivés de formule (I),
X représentant ici un atome d'hydrogène,
Z formant avec Y une liaison π ,
dont, si on le désire, on sépare les isomères cis-trans par une technique choisie parmi cristallisation ou chromatographie,
et que l'on salifie, si on le désire, par un acide pharmaceutiquement acceptable,
qui, si on le désire, est soumis à une réaction d'hydrogénation catalytique préférentiellement à température et pression ambiantes, et en présence de Nickel de Raney en milieu d'alcool aliphatique inférieur ou de dioxanne pour obtenir un dérivé de formule (I/D) :

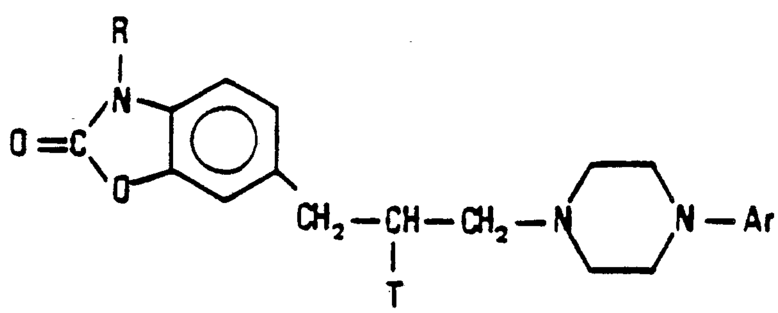

(I/D)

dans laquelle :
R, T et Ar ont la même signification que dans la formule (I),
X, Y et Z représentant chacun simultanément un atome d'hydrogène.
dont on sépare le cas échéant les isomères lorsque T ne représente pas un atome d'hydrogène,
et que l'on salifie éventuellement par un acide pharmaceutiquement acceptable.

3/ Procédé de préparation des dérivés de formule (I/D), selon la revendication 2 à partir des dérivés de formule (IX) :

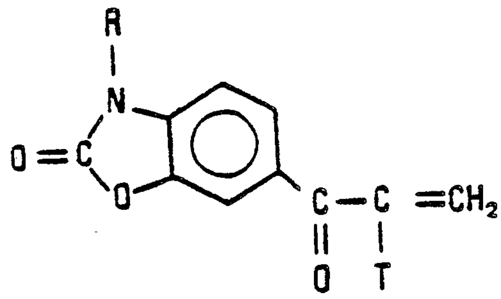

(IX)

dans laquelle :
R et T ont la même signification que dans la formule (I),
que l'on traite par un agent d'halogénation pour obtenir un dérivé de formule (IV) :

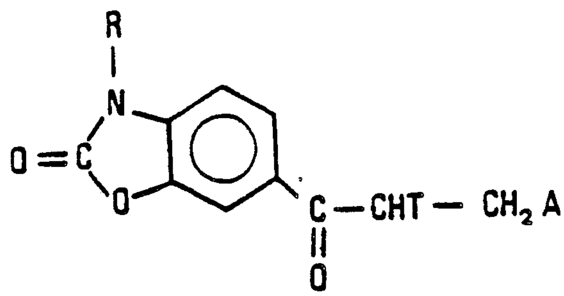

(IV)

dans laquelle R et T ont la même signification que dans la formule (I) et A la même signification que dans la formule (III),
dont on sépare si on le désire, les isomères, lorsque H ne représente pas un atome d'hydrogène,
que l'on soumet à réduction par un trialkylsilane, pour conduire à un dérivé de formule (V) :

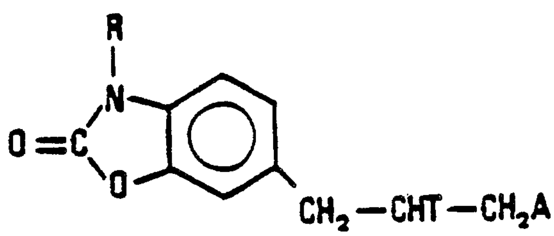

(V)

dans laquelle R et T ont la même signification que dans la formule (I), et A la même signification que dans la formule (III),
que l'on soumet à l'action d'une aryl - 1 pipérazine de formule (VI) :

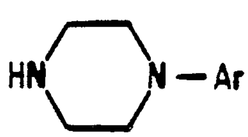

(VI)

dans laquelle Ar a la même signification que dans la formule (I), dans un solvant préférentiellement choisi parmi acétone, acétonitrile, acétate d'éthyle, alcool aliphatique inférieur, dioxanne, benzène, toluène, à une température comprise entre la température ambiante et la température d'ébullition du solvant choisi en présence d'un excès de l'amine choisie ou d'un capteur de l'hydracide formé tel que la triéthylamine,
pour conduire à un dérivé de formule (I/D) selon la revendication 9,
et que l'on salifie, si on le désire par un acide pharmaceutiquement acceptable.

4/ Procédé selon l'une des revendications 1 à 3 de préparation des composés de formule (I) dans lesquels X et Y simultanément représentent ensemble un atome d'oxygène, ainsi que leurs sels d'addition à

un acide pharmaceutiquement acceptable.

5/ Procédé selon l'une des revendications 1 à 3 de préparation des composés de formule (I) dans lesquels X représente un atome d'hydrogène et Y un groupement hydroxyle, leurs énantiomères, épimères, diastéréoisomères, ainsi que leurs sels d'addition à un acide pharmaceutiquement acceptable.

6/ Procédé selon l'une des revendications 1 à 3 de préparation des composés de formule (I) dans lesquels X et Y représentent simultanément chacun un atome d'hydrogène, ainsi que leurs sels d'addition à un acide pharmaceutiquement acceptable.

7/ Procédé selon l'une des revendications 1 à 3 de préparation des composés de formule (I) dans lesquels Y forme avec Z une liaison π, leurs isomères ainsi que leurs sels d'addition à un acide pharmaceutiquement acceptable.

8/ Procédé selon l'une des revendications 1 à 3 de préparation des composés de formule (I) dans lesquels Ar représente le groupement phényle, leurs énantiomères, épimères, diastéréoisomères ainsi que leurs sels d'addition à un acide pharmaceutiquement acceptable.

9/ Procédé selon l'une des revendications 1 à 3 de préparation des composés de formule (I) dans lesquels Ar représente un groupement phényle substitué par un atome d'halogène, un groupement alkoxy inférieur ou un groupement trifluorométhyle, leurs énantiomères, épimères, diastéréoisomères ainsi que leurs sels d'addition à un acide pharmaceutiquement acceptable.

10/ Procédé selon l'une des revendications 1 à 3 de préparation des composés de formule (I) dans lesquels Ar représente un radical pyridyle - 2 ou pyrimidinyle - 2, leurs énantiomères, épimères, diastéréoisomères ainsi que leurs sels d'addition à un acide pharmaceutiquement acceptable.

Revendications pour l'Etat contractant suivant: ES

1/ Procédé de préparation des composés de formule générale (I) :

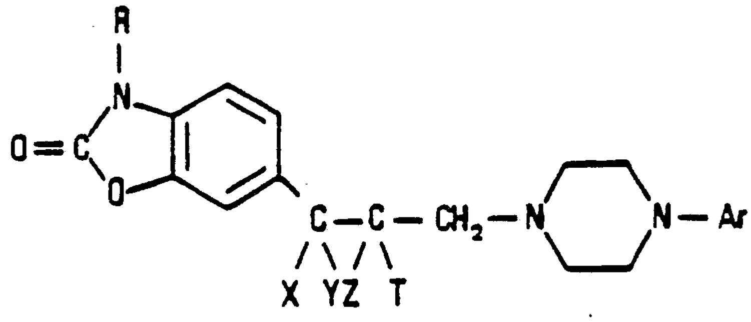

(I)

dans laquelle :

X représente un atome d'hydrogène,

Y représente un atome d'hydrogène ou un groupement hydroxyle ou bien X et Y représentent ensemble un atome d'oxygène,

T représente un atome d'hydrogène ou un groupement alcoyle inférieur,

Z représente un atome d'hydrogène ou bien Z forme avec Y une liaison π, et dans ce cas X représente un atome d'hydrogène,

R représente un atome d'hydrogène ou un groupement alcoyle inférieur,

Ar représente un groupement aryle, ou hétéroaryle, ou alkyl inférieur aryle, éventuellement substitué par un atome d'halogène, un groupement alcoyle ou alcoyloxy inférieur, eux-mêmes éventuellement substitués par un ou plusieurs atomes d'halogène,

leurs énantiomères, épimères, diastéréoisomères,

ainsi que leurs sels d'addition à un acide pharmaceutiquement acceptable,

caractérisé en ce que l'on utilise comme matière première un dérivé de formule (II) :

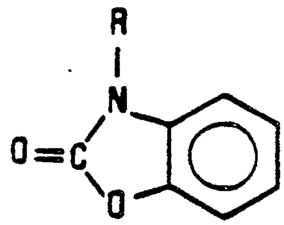

(II)

dans laquelle, R a la même signification que dans la formule (I),
que l'on soumet à l'action d'un chlorure d'acide de formule (III) :

**Cl-CO-HC -$CH_2$-A** (III)
**T**

dans laquelle, T a la même signification que dans la formule (I), A représentant un atome d'halogène,
ou bien de l'anhydride d'acide correspondant,
en présence de chlorure d'aluminium en milieu de diméthyl formamide,
pour obtenir un dérivé de formule (IV) :

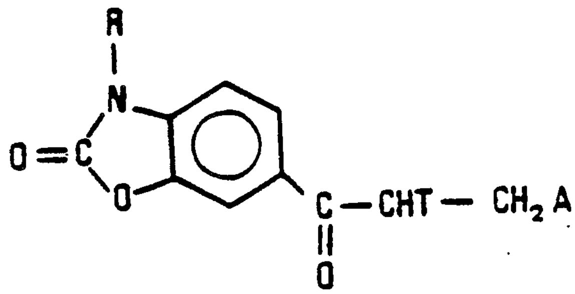

(IV)

dans laquelle R et T ont la même signification que dans la formule (I) et A la même signification que dans la formule (III),
qui, si on le désire, est soumis à réduction par un trialkylsilane en milieu acide, pour conduire à un dérivé de formule (V) :

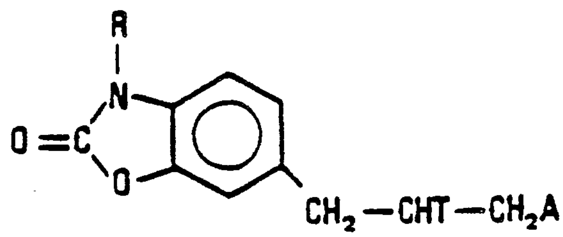

(V)

dans laquelle R et T ont la même signification que dans la formule (I), et A la même signification que dans la formule (III),
le dérivé de formule (IV), ou le dérivé de formule (V), selon la formule du dérivé de formule (I) que l'on souhaite obtenir, étant alors soumis à l'action d'une aryl - 1 pipérazine de formule (VI) :

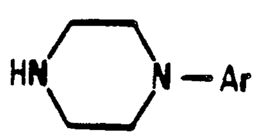

(VI)

dans laquelle Ar a la même signification que dans la formule (I),
dans un solvant préférentiellement choisi parmi acétone, acétonitrile, acétate d'éthyle, alcool aliphatique inférieur, dioxanne, benzène, toluène, à une température comprise entre la température ambiante et la

température d'ébullition du solvant choisi, en présence d'un excès de l'amine choisie ou d'un capteur de l'hydracide formé tel que la triéthylamine,
pour conduire à un dérivé de formule (I/A) :

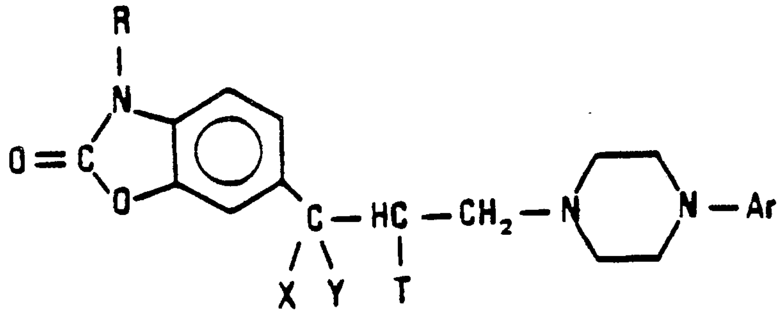

(I/A)

dans laquelle, selon que la matière première utilisée est un dérivé de formule (IV) ou (V),
X et Y représentent ensemble un atome d'oxygène, ou bien X et Y représentent tous deux simultanément un atome d'hydrogène,
R, Ar et T ayant la même signification que dans la formule (I),
qui, si on le désire, est salifié par un acide pharmaceutiquement acceptable ou qui peut, lorsque X et Y représentent ensemble un atome d'oxygène, si on le désire, être soumis,
* ou bien, à un agent d'hydrogénation choisi parmi un hydrure mixte de métal alcalin, comme par exemple le borohydrure de sodium, ou un cyanohydrure mixte de métal alcalin, comme le cyano borohydrure de sodium, préférentiellement en milieu d'alcool aliphatique inférieur, pour conduire à un dérivé de formule (I/B) - majoritairement sous la configuration thréo lorsque T ne représente pas un atome d'hydrogène - :

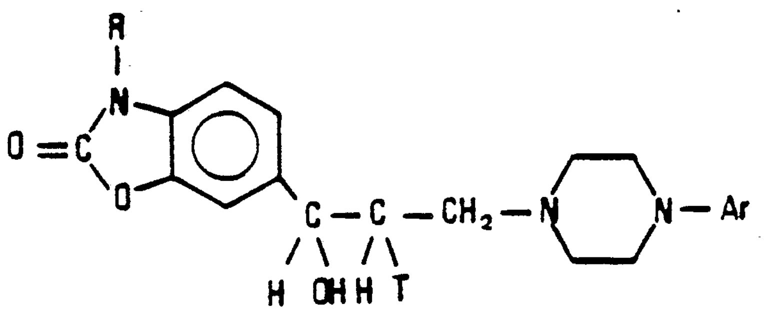

(I/B)

cas particulier des dérivés de formule (I), dans laquelle :
R, T et Ar ont la même signification que dans les dérivés de formule (I),
X représentant ici un atome d'hydrogène,
Y un groupement hydroxyle et Z un atome d'hydrogène,
dont, on sépare, si on le désire, les isomères et/ou que l'on salifie par un acide pharmaceutiquement acceptable,
* ou bien à hydrogénation catalytique, sous chauffage et pression dans un solvant choisi parmi alcool aliphatique inférieur ou dioxanne, pour conduire à un dérivé de formule (I/B) - essentiellement sous la configuration érythro lorsque T ne représente pas un atome d'hydrogène - dont on sépare, si on le désire, les isomères et que l'on salifie le cas échéant par un acide pharmaceutiquement acceptable,
dérivé de formule (I/B) qui, quelque soit le procédé selon lequel il a été obtenu peut être, si on le désire, traité par un agent déshydratant,
préférentiellement choisi parmi les hydracides, pour conduire à un dérivé de formule (I/C) :

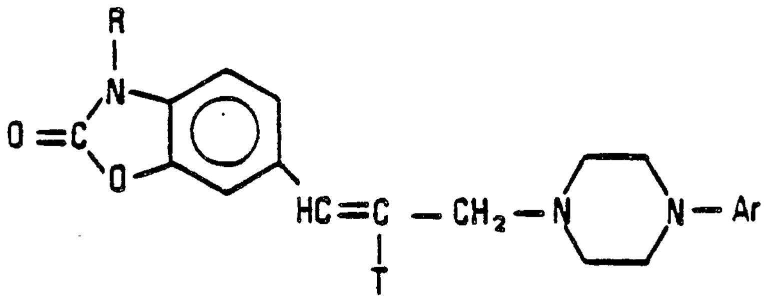

(I/C)

cas particulier de dérivés de formule (I), dans laquelle :

R, T et Ar ont la même signification que dans les dérivés de formule (I),

X représentant ici un atome d'hydrogène,

Z formant avec Y une liaison π ,

dont, on sépare, si on le désire, les isomères cis-trans par une technique choisie parmi cristallisation ou chromatographie,

et qui, si on le souhaite, peut être salifié par un acide pharmaceutiquement acceptable.

2/ Procédé de préparation des dérivés de formule (I) pour lesquels R représente un groupement alcoyle inférieur caractérisé en ce que l'on acyle un dérivé de formule (II) :

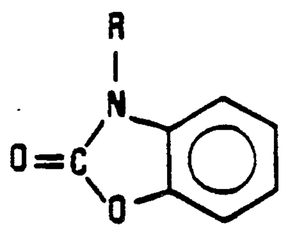

(II)

dans laquelle R représente un groupement alcoyle inférieur par un acide de formule (VII) :

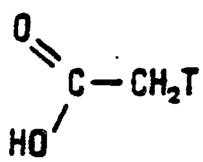

(VII)

dans laquelle T a la même signification que dans la formule (I) ou le chlorure ou l'anhydride de l'acide correspondant en présence d'acide polyphosphonique,

pour obtenir un dérivé de formule (VIII) :

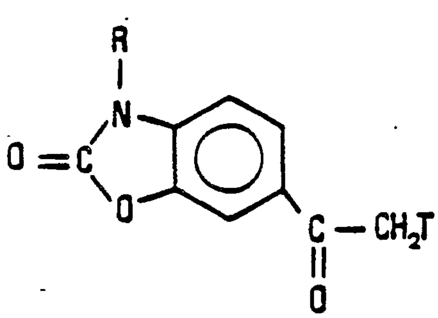

(VIII)

dans laquelle :

R représente un groupement alcoyle inférieur, et T a la même signification que dans la formule (I),

que l'on traite ensuite,
* ou bien, selon les conditions de la réaction de Mannich, bien connues de l'homme de l'Art, en présence de trioxyméthylène et de l'aryl pipérazine choisie de formule (VI) :

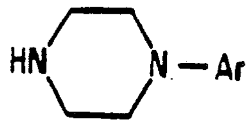

(VI)

dans laquelle, Ar a la même signification que dans la formule (I),
pour obtenir un dérivé de formule (I/A1) :

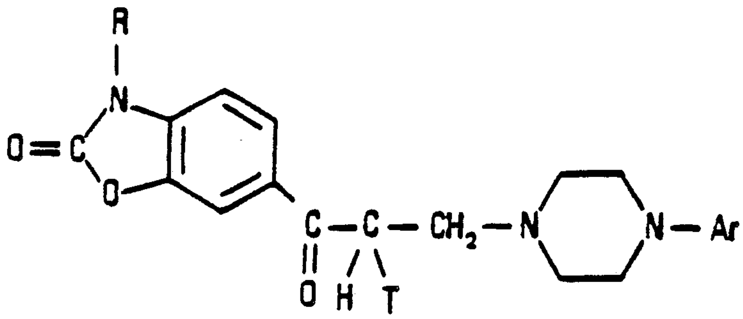

(I/A1)

cas particulier des dérivés de formules (I/A) et (I), dans laquelle :
R représente un groupement alcoyle inférieur, et T et Ar ont la même signification que dans la formule (I),
X et Y représentant ici simultanément un atome d'oxygène et Z un atome d'hydrogène,
* ou bien par le bisdiméthylamino méthane en milieu d'anhydride acétique pour obtenir un produit de formule générale (IX) :

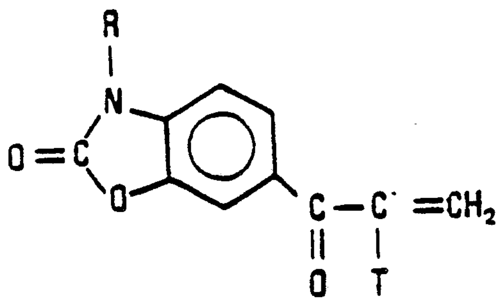

(IX)

dans laquelle :
T a la même signification que dans la formule (I) et R représente un groupement alcoyle inférieur,
que l'on traite par une amine de formule (VI), dans un solvant polaire à une température comprise entre la température ambiante et la température d'ébullition du milieu réactionnel,
pour conduire à un dérivé de formule (I/A1) précédemment défini,
qui, lorsque T ne représente pas un atome d'hydrogène peut être, si on le désire, séparé en ses énantiomères, que l'on salifie, si on le désire par un acide pharmaceutiquement acceptable et qui,
si on le souhaite, peut être soumis,
* ou bien préférentiellement en milieu d'alcool aliphatique inférieur à un agent d'hydrogénation préférentiellement un hydrure mixte de métal alcalin ou un cyanohydrure mixte de métal alcalin comme par exemple le borohydrure de sodium, ou le cyanoborohydrure de sodium,
pour conduire à un dérivé de formule (I/B) majoritairement sous la configuration thréo (lorsque T ne représente pas un atome d'hydrogène) :

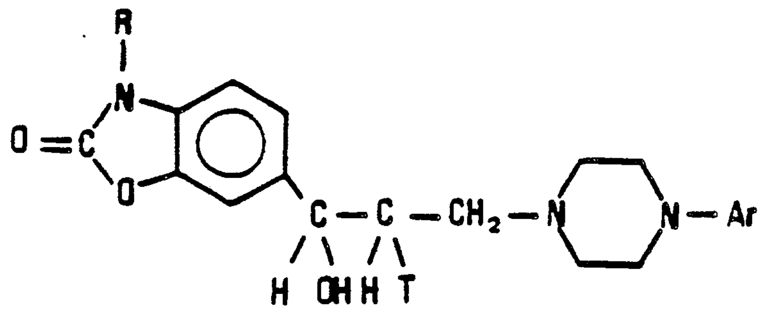

(I/B)

cas particulier des dérivés de formule (I), dans laquelle :
R, T et Ar ont la même signification que dans les dérivés de formule (I),
X représentant ici un atome d'hydrogène,
Y un groupement hydroxyle et Z un atome d'hydrogène,
dont on sépare, si on le désire, les isomères et que l'on peut salifier par un acide pharmaceutiquement acceptable,
* ou bien à hydrogénation catalytique dans un solvant choisi parmi alcool, aliphatique inférieur ou dioxanne pour conduire à un dérivé de formule (I/B) essentiellement sous la configuration érythro -lorsque T ne représente pas un atome d'hydrogène - dont on sépare, si on le désire, les énantiomères et que l'on salifie le cas échéant par un acide pharmaceutiquement acceptable,
dérivé de formule (I/B) que, le cas échéant on soumet à un agent déshydratant choisi parmi les hydracides pour conduire à un dérivé de formule (I/C) :

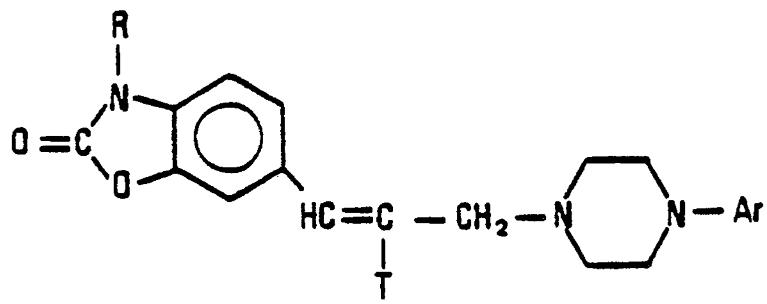

(I/C)

cas particulier de dérivés de formule (I), dans laquelle :
R, T et Ar ont la même signification que dans les dérivés de formule (I),
X représentant ici un atome d'hydrogène,
Z formant avec Y une liaison π ,
dont, si on le désire, on sépare les isomères cis-trans par une technique choisie parmi cristallisation ou chromatographie,
et que l'on salifie, si on le désire, par un acide pharmaceutiquement acceptable,
qui, si on le désire, est soumis à une réaction d'hydrogénation catalytique préférentiellement à température et pression ambiantes, et en présence de Nickel de Raney en milieu d'alcool aliphatique inférieur ou de dioxanne pour obtenir un dérivé de formule (I/D) :

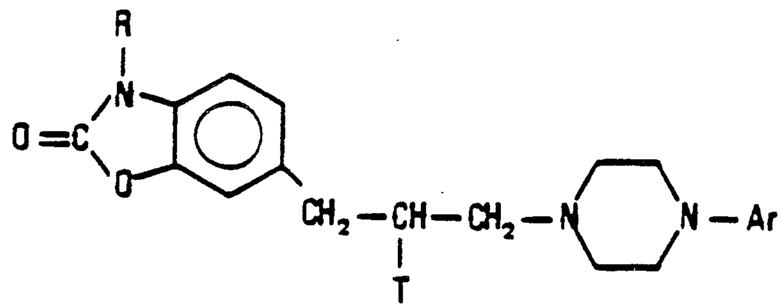

(I/D)

dans laquelle :
R, T et Ar ont la même signification que dans la formule (I),
X, Y et Z représentant chacun simultanément un atome d'hydrogène.
dont on sépare le cas échéant les isomères lorsque T ne représente pas un atome d'hydrogène,
et que l'on salifie éventuellement par un acide pharmaceutiquement acceptable.

3/ Procédé de préparation des dérivés de formule (I/D), selon la revendication 2 à partir des dérivés de formule (IX) :

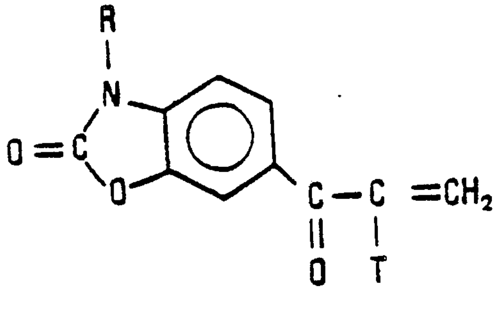

(IX)

dans laquelle :
R et T ont la même signification que dans la formule (I),
que l'on traite par un agent d'halogénation pour obtenir un dérivé de formule (IV) :

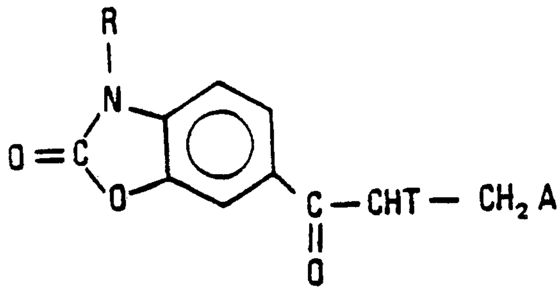

(IV)

dans laquelle R et T ont la même signification que dans la formule (I) et A la même signification que dans la formule (III),
dont on sépare si on le désire, les isomères, lorsque H ne représente pas un atome d'hydrogène,
que l'on soumet à réduction par un trialkylsilane, pour conduire à un dérivé de formule (V) :

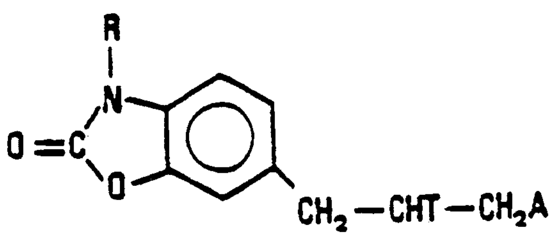

(V)

dans laquelle R et T ont la même signification que dans la formule (I), et A la même signification que dans la formule (III),
que l'on soumet à l'action d'une aryl - 1 pipérazine de formule (VI) :

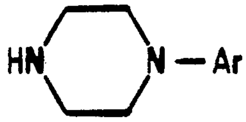

(VI)

dans laquelle Ar a la même signification que dans la formule (I), dans un solvant préférentiellement choisi parmi acétone, acétonitrile, acétate d'éthyle, alcool aliphatique inférieur, dioxanne, benzène, toluène, à une température comprise entre la température ambiante et la température d'ébullition du solvant choisi en présence d'un excès de l'amine choisie ou d'un capteur de l'hydracide formé tel que la triéthylamine, pour conduire à un dérivé de formule (I/D) selon la revendication 9, et que l'on salifie, si on le désire par un acide pharmaceutiquement acceptable.

4/ Procédé selon l'une des revendications 1 à 3 de préparation des composés de formule (I) dans lesquels X et Y simultanément représentent ensemble un atome d'oxygène, ainsi que leurs sels d'addition à un acide pharmaceutiquement acceptable.

5/ Procédé selon l'une des revendications 1 à 3 de préparation des composés de formule (I) dans lesquels X représente un atome d'hydrogène et Y un groupement hydroxyle, leurs énantiomères, épimères, diastéréoisomères, ainsi que leurs sels d'addition à un acide pharmaceutiquement acceptable.

6/ Procédé selon l'une des revendications 1 à 3 de préparation des composés de formule (I) dans lesquels X et Y représentent simultanément chacun un atome d'hydrogène, ainsi que leurs sels d'addition à un acide pharmaceutiquement acceptable.

7/ Procédé selon l'une des revendications 1 à 3 de préparation des composés de formule (I) dans lesquels Y forme avec Z une liaison $\pi$ , leurs isomères ainsi que leurs sels d'addition à un acide pharmaceutiquement acceptable.

8/ Procédé selon l'une des revendications 1 à 3 de préparation des composés de formule (I) dans lesquels Ar représente le groupement phényle, leurs énantiomères, épimères, diastéréoisomères ainsi que leurs sels d'addition à un acide pharmaceutiquement acceptable.

9/ Procédé selon l'une des revendications 1 à 3 de préparation des composés de formule (I) dans lesquels Ar représente un groupement phényle substitué par un atome d'halogène, un groupement alkoxy inférieur ou un groupement trifluorométhyle, leurs énantiomères, épimères, diastéréoisomères ainsi que leurs sels d'addition à un acide pharmaceutiquement acceptable.

10/ Procédé selon l'une des revendications 1 à 3 de préparation des composés de formule (I) dans lesquels Ar représente un radical pyridyle - 2 ou pyrimidinyle - 2, leurs énantiomères, épimères, diastéréoisomères ainsi que leurs sels d'addition à un acide pharmaceutiquement acceptable.

11/ Procédé selon l'une des revendications 1 à 3 de préparation des composés de formule (I) qui est la méthyl - 3 {[(fluoro - 4 phényl) -4 pipérazinyl - 1] - 3 oxo - 1 propyl} - 6 benzoxazolinone, ainsi que leurs sels d'addition à un acide pharmaceutiquement acceptable.

12/ Procédé selon l'une des revendications 1 à 3 de préparation des composés de formule (I) qui est la méthyl - 3 {[(méthoxy - 2 phényl) -4 pipérazinyl - 1] - 3 oxo - 1 propyl} - 6 benzoxazolinone, ainsi que leurs sels d'addition à un acide pharmaceutiquement acceptable.

Office européen des brevets

# RAPPORT DE RECHERCHE EUROPEENNE

Numero de la demande

EP 89 40 2736

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| D,Y | EP-A-0 110 781 (RIOM LABORATORIES-C.E.R.M.)<br>* Revendications *<br>--- | 1,14,15 | C 07 D 263/58<br>A 61 K 31/42<br>C 07 D 413/12 |
| Y | EUROPEAN JOURNAL OF MEDICINAL CHEMISTRY-CHIMICA THERAPEUTICA, vol. 11, no. 1, janvier-février 1976, pages 33-42, Paris, FR; J.-C. CAZIN et al.: "Etude de chimique et pharamacodynamique B-amino-cétones de structure benzoxazolinonique"<br>* En entier *<br>--- | 1,14,15 | |
| D,Y | EP-A-0 049 203 (CERM)<br>* Revendications *<br>----- | 1,14,15 | |

DOMAINES TECHNIQUES RECHERCHES (Int. Cl.5)

C 07 D 263/00
C 07 D 413/00

Le présent rapport a été établi pour toutes les revendications

| Lieu de la recherche | Date d'achèvement de la recherche | Examinateur |
|---|---|---|
| LA HAYE | 16-11-1989 | HENRY J.C. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)